(19) Europäisches Patentamt
European Patent Office
Office européen des brevets

(11) EP 2 289 310 B1

(12) **EUROPÄISCHE PATENTSCHRIFT**

(45) Veröffentlichungstag und Bekanntmachung des Hinweises auf die Patenterteilung:
**28.03.2012 Patentblatt 2012/13**

(51) Int Cl.:
***A01H 3/04*** *(2006.01)* ***C12Q 1/68*** *(2006.01)*
***A01N 43/72*** *(2006.01)*

(21) Anmeldenummer: **10175325.9**

(22) Anmeldetag: **10.11.2006**

(54) **Wirkstoffe zur Ertragssteigerung in Pflanzen gegenüber Trocken- und/oder Hitzestress**

Active ingredients for increasing a plant's yield in relation to drought and/or heat-stress

Substances pour augmenter le rendement d'une plante en condition de secheresse et/ou stress thermique

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **29.11.2005 DE 102005057250**

(43) Veröffentlichungstag der Anmeldung:
**02.03.2011 Patentblatt 2011/09**

(62) Dokumentnummer(n) der früheren Anmeldung(en) nach Art. 76 EPÜ:
**06818467.0 / 1 956 885**

(73) Patentinhaber: **Bayer CropScience AG**
**40789 Monheim (DE)**

(72) Erfinder:
- **Schulz, Arno**
  **65817, Eppstein (DE)**
- **Bartsch, Klaus**
  **61462, Königstein (DE)**
- **Krämer, Hansjörg**
  **65719, Hofheim (DE)**
- **Hills, Martin**
  **65510, Idstein (DE)**
- **Hacker, Erwin**
  **65239, Hochheim (DE)**
- **Rosinger, Chris**
  **65719, Hofheim (DE)**

(56) Entgegenhaltungen:
**WO-A-00/28055**
**WO-A-2004/092398**
**WO-A1-2005/048710**
**DD-A1- 277 832**
**DE-A1- 10 127 328**
**US-A1- 2001 001 095**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die Verwendung von Safenern zur Erhöhung der Pflanzentoleranz gegenüber abiotischen Stressoren wie Hitze und Trockenheit durch Expressionssteigerung pflanzenendogener Proteine.

Es ist bekannt, dass Pflanzen auf natürliche Stressbedingungen, wie beispielsweise Kälte, Hitze, Trockenheit, Verwundung, Pathogenbefall (Viren, Bakterien, Pilze, Insekten) etc. aber auch auf Herbizide mit spezifischen oder unspezifischen Abwehrmechanismen reagieren [Pflanzenbiochemie, S. 393-462 , Spektrum Akademischer Verlag, Heidelberg, Berlin, Oxford, Hans W. Heldt, 1996.; Biochemistry and Molecular Biology of Plants, S. 1102-1203, American Society of Plant Physiologists, Rockville, Maryland, eds. Buchanan, Gruissem, Jones, 2000].

In Pflanzen sind zahlreiche Proteine und die sie codierenden Gene bekannt, die an Abwehrreaktionen gegen abiotischen Stress (z.B. Kälte, Hitze, Trockenheit, Salz) beteiligt sind. Diese gehören teilweise zu Signaltransduktionsketten (z.B. Transkriptionsfaktoren, Kinasen, Phosphatasen) oder bewirken eine physiologische Antwort der Pflanzenzelle (z.B. Ionentransport, Entgiftung reaktiver Sauerstoff-Spezies). Zu den Signalkettengenen der abiotischen Stressreaktion gehören u.a. Transkriptionsfaktoren der Klassen DREB und CBF (Jaglo-Ottosen et al., 1998, Science 280: 104-106). An der Reaktion auf Salzstress sind Phosphatasen vom Typ ATPK und MP2C beteiligt. Ferner wird bei Salzstress häufig die Biosynthese von

Osmolyten wie Prolin oder Sucrose aktiviert. Beteiligt sind hier z.B. die Sucrose-Synthase und Prolin-Transporter (Hasegawa et al., 2000, Annu Rev Plant Physiol Plant Mol Biol 51: 463-499). Die Stressabwehr der Pflanzen gegen Kälte und Trockenheit benutzt z.T. die gleichen molekularen Mechanismen. Bekannt ist die Akkumulation von sogenannten Late Embryogenesis Abundant Proteins (LEA-Proteine), zu denen als wichtige Klasse die Dehydrine gehören (Ingram and Bartels, 1996, Annu Rev Plant Physiol Plant Mol Biol 47: 277-403, Close, 1997, Physiol Plant 100: 291-296). Es handelt sich dabei um Chaperone, die Vesikel, Proteine und Membranstrukturen in gestressten Pflanzen stabilisieren (Bray, 1993, Plant Physiol 103: 1035-1040). Außerdem erfolgt häufig eine Induktion von Aldehyd-Deydrogenasen , welche die bei oxidativem Stress entstehenden reaktiven Sauerstoff-Spezies (ROS) entgiften (Kirch et al., 2005, Plant Mol Biol 57: 315-332). Heat Shock Faktoren (HSF) und Heat Shock Proteine (HSP) werden bei Hitzestress aktiviert und spielen hier als Chaperone eine ähnliche Rolle wie die Dehydrine bei Kälte- und Trockenstress (Yu et al., 2005, Mol Cells 19: 328-333).

Die meisten der beschriebenen molekularen Mechanismen werden durch Induktion der Genexpression aktiviert. Dadurch ergibt sich die interessante Möglichkeit spezifische Stressantworten von Pflanzen mit Hilfe der Transkriptom-Analyse, z.B. durch Gene Expression Profiling (GEP) mit DNA Microarrays oder vergleichbaren Techniken zu charakterisieren (Rensink et al., 2005, Genome 48: 598-605, Cheong et al., 2002, Plant Physiology 129: 661-677). Auf diese Weise lassen sich spezifische Stress-reaktive Genexpressionsmuster erfassen und miteinander vergleichen.

Es ist weiter bekannt, dass chemische Substanzen die Toleranz von Pflanzen gegen abiotischen Stress erhöhen können. Derartige Substanzen werden dabei entweder durch Saatgut-Beizung, durch Blattspritzung oder durch Bodenbehandung appliziert. So wird eine Erhöhung der abiotischen Stresstoleranz von Kulturpflanzen durch Behandlung mit Elicitoren der Systemic Acquired Resistance (SAR) oder Abscisinsäure-Derivaten beschrieben (Schading and Wei, WO-200028055, Abrams and Gusta, US-5201931, Churchill et al., 1998, Plant Growth Regul 25: 35-45). Auch bei Anwendung von Fungiziden, insbesondere aus der Gruppe der Strobilurine werden ähnliche Effekte beobachtet, die häufig auch mit einer Ertragssteigerung einhergehen (Draber et al., DE-3534948, Bartlett et al., 2002, Pest Manag Sci 60: 309).

Desweiteren wurden Effekte von Wachstumsregulatoren auf die Stresstoleranz von Kulturpflanzen beschrieben (Morrison and Andrews, 1992, J Plant Growth Regul 11: 113-117, RD-259027). Bei osmotischem Stress ist eine Schutzwirkung durch Applikation von Osmolyten wie z.B. Glycinbetain oder deren biochemischen Vorstufen, z.B. Cholin-Derivate beobachtet worden (Chen et al., 2000, Plant Cell Environ 23: 609-618, Bergmann et al., DE-4103253). Auch die Wirkung von Antioxidantien wie z.B Naphtole und Xanthine zur Erhöhung der abiotischen Stresstoleranz in Pflanzen wurde bereits beschrieben (Bergmann et al., DD-277832, Bergmann et al., DD-277835). Die molekularen Ursachen der Anti-Stress-Wirkung dieser Substanzen sind jedoch weitgehend unbekannt.

Somit ist bekannt, dass Pflanzen über mehrere endogene Reaktionsmechanismen verfügen, die eine wirksame Abwehr gegenüber verschiedensten Schadorganismen und/oder natürlichem abiotischen Stress bewirken können. Eine Voraussage darüber, welche Abwehrreaktionen gezielt durch Anwendung von Wirkstoffen hervorgerufen oder moduliert werden können, war bisher jedoch nicht bekannt.

Es besteht somit ein Bedarf nach einer Methode zum gezielten Auffinden molekularer Aktivatoren pflanzenendogener Abwehrmechanismen gegenüber abiotischem Stress (wie beispielsweise Hitze, Kälte, Trockenheit, Salinität, sowie Säuren-/und Basenbelastung) wodurch neuartige Wirkstoffe aufgefunden, neue Eigenschaften bekannter, aber andersartig wirkender Wirkstoffe identifiziert, oder aber bereits bekannte Moleküle oder Leitstrukturen auf die Verwendung als Induktoren der pflanzenendogenen Abwehrmechanismen gegenüber abiotischen Stressoren optimiert werden können.

Definitionen nachfolgend verwendeter Begriffe

Der Begriff "Blast-Analysen" (Blast = Basic Local Alignment Search Tool)", wie hier verwendet, beschreibt die Verwendung geeigneter Computer-Programme zur Klassifikation und zum Auffinden potentiell homologer Sequenzen (Altschul et al., J. Mol. Biol. 1990, 215: 403-410), wobei ein Vergleich (Alignment) zwischen einer Suchsequenz (query sequence) und allen Sequenzen einer oder mehrerer Datenbanken unter Vorgabe einer gewünschten Übereinstimmung in Form eines "Signifkanz-Kriteriums" (scoring function), erfolgt (R. Rauhut, Bioinformatik, S 38-107, Verlag Wiley-VCH Verlag GmbH, Weinheim, 2001).

Der Begriff "cDNA" (complementary DNA), wie hier verwendet, beschreibt einen DNA-Einzelstrang, der zu einer RNA komplementär, und der durch eine enzymatische reverse Transkription *in vitro* synthetisiert wird. Die cDNA kann entweder der gesamten RNA-Länge entsprechen, oder aber nur eine Teilsequenz der als Matrix dienenden RNA darstellen.

Der Begriff "Cluster-Analyse", wie hier verwendet, bedeutet die Zusammenfassung der ermittelten Einzeldaten mittels eines hierfür entwickelten Computerprogramms, wobei Gruppen von Genen, die für Proteine mit ähnlicher Funktion kodieren, oder aber Gene mit ähnlichem Expressionsmuster zusammenfassend dargestellt werden. Hierdurch wird eine hierarchische Minimierung des komplexen Datenmusters erreicht, die in Form eines Dendrograms dargestellt werden kann. Die Cluster-Analyse ermöglicht die klassifizierende Bewertung der erhaltenen Datensätze, die deutlich über die bloße Akkumulierung beziehungsloser Daten hinausgeht.

Unter den Begriffen "DNA-Chip" und "DNA-Microarray", die hier synonym verwendet werden, wird ein Träger bezeichnet dessen Grundmaterial beispielsweise aus Glas oder Nylon besteht, auf dessen Grundmaterial DNA-Fragmente fixiert sind, wobei die Aufbringung der DNA beispielsweise durch (a) ein photolithographisches Verfahren (DNA wird direkt auf dem Arrayträger synthetisiert), (b) ein Microspotting-Verfahren (extern synthetisierte Oligonukleotide oder PCR-Produkte werden auf den Träger appliziert und kovalent gebunden), oder (c) durch eine Microspraying-Verfahren (extern synthetisierte Oligonukleotide oder PCR-Produkte werden mit einem Tintenstrahldrucker berührungsfrei auf den Träger gesprüht) erfolgen kann (R. Rauhut, Bioinformatik, S 197-199, Verlag Wiley-VCH Verlag GmbH, Weinheim, 2001). Ein DNA-Chip, der genomische Sequenzen eines Organismus representiert, wird als "genomischer DNA-Chip" bezeichnet. Die Auswertung der mit Hilfe dieser DNA-Chips" erhaltenen Messwerte wird als "DNA-Chip-Analyse" bezeichnet.

Der Begriff "DNA-Chip-Hybridisierung", wie hier verwendet, bedeutet die Paarung zweier einzelsträngiger, komplementärer Nukleinsäuremoleküle, wobei eines der basenpaarenden Molekülpartner als DNA (Desoxribonukleinsäure) auf dem DNA-Chip in bevorzugt kovalent gebundener Form lokalisiert ist, während der andere in Form der RNA (Ribonukleinsäure) oder der hierzu korrespondierenden cDNA (komplementäre DNA) in Lösung vorliegt. Die Hybridisierung der gebundenen und nicht gebundenen Nukleinsäuren erfolgt auf dem DNA-Chip in wässriger Pufferlösung, gegebenenfalls unter zusätzlich denaturierenden Bedingungen, wie beispielsweise in Gegenwart von Dimethylsulfoxid, bei Temperaturen von 30-60˚C, bevorzugt 40-50˚C, besonders bevorzugt bei 45˚C für 10-20 Stunden, bevorzugt für 14-18 Stunden, besonders bevorzugt für 16 Stunden unter ständiger Bewegung. Die Hygbridisierungsbedingungen können konstant beispielsweise in einem Hybridisierungsofen realisiert werden. Standardmäßig werden in einem solchen Hybridisierungsofen Bewegungen von 60 rpm (rounds per minute, Umdrehungen pro Minute) realisiert.

Die mit dem Begriff "EST-Sequenz" (Expressed Sequence Tag) bezeichnete Nukleinsäuresequenz, wie hier verwendet, bedeutet eine kurze Sequenz von 200-500 Basen oder Basenpaaren.

Die synonym gebrauchten Begriffe "Expressionsmuster", "Induktionsmuster" bzw. "Expressionsprofil", wie hier verwendet, beschreiben die zeitlich differenzierte und/oder gewebespezifische Expression der pflanzlichen mRNA, wobei das Muster direkt durch die erzeugte Intensität des Hybridisierungssignals der aus der Pflanze erhaltenen RNA oder deren korrespondierender cDNA mit Hilfe der DNA-Chip-Technologie erhalten wird. Die gemessenen "Induktionswerte" ergeben sich durch direkte Verrechnung mit den korrespondierenden Signalen, die durch Verwendung eines synonymen Chips unter Hybridisierung mit einer nicht behandelten/ gestressten Kontrollpflanze erhalten werden.

Der Begriff "Expressionszustand" der durch das vorgenommene "Gene Expression Profiling" erhalten wird, wie hier verwendet, beschreibt die gesamte erfasste Transkriptionsaktivität zellulärer Gene, die mit Hilfe eines DNA-Chips gemessen wird.

Der Begriff "Gesamt-RNA", wie hier verwendet, beschreibt die aufgrund des angewendeten Aufschlussverfahrens mögliche Repräsentanz verschiedener pflanzenendogener RNA-Gruppen, die in einer Pflanzenzelle vorliegen können, wie beispielsweise, cytoplasmatische rRNA (ribosomale RNA), cytoplasmatische tRNA (transfer RNA), cytoplasmatische mRNA (messenger RNA), sowie deren jeweilige nucleäre Vorläufer, ctRNA (chloroplastidäre RNA) und mtRNA (mitochondriale RNA), sie umfasst aber auch RNA-Moleküle, die von exogenen Organismen stammen können, wie beispielsweise von Viren, oder parasitierenden Bakterien und Pilzen.

Der Begriff "Nutzpflanzen", wie hier verwendet, bezeichnet Kulturpflanzen, die als Pflanzen für die Gewinnung von Nahrungsmitteln, Futtermitteln oder für technische Zwecke eingesetzt werden.

Der Begriff "Safener", wie hier verwendet, bezeichnet eine chemische Verbindung, die nicht pflanzenendogenen

Ursprungs ist, und die die phytotoxischen Eigenschaften eines Pestizids gegenüber Nutzpflanzen aufhebt oder verringert, ohne dass die pestizide Wirkung gegenüber Schadorganismen, wie beispielsweise Unkräutern, Bakterien, Viren und Pilzen wesentlich vermindert wird.

Safener, die neben ihrer eigentlich bekannten Funktion ebenfalls zur Erhöhung der Toleranz gegenüber abiotischen Stressoren beitragen, sind vorzugsweise ausgewählt aus der nachstehend definierten Gruppe, wobei die Auswahl je nach abiotischem Stressor unterschiedlich erfolgen kann, die Verwendung nur eines einzelnen Saferners oder aber mehrerer Safener aus der selben oder aus verschiedenen Gruppen erfolgen kann:

a) Verbindungen der Formeln (I) bis (III),

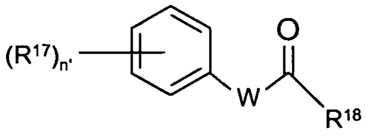

(I)

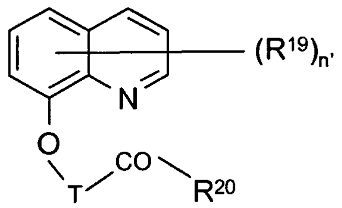

(II)

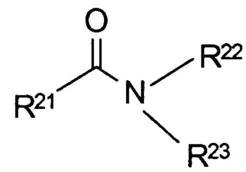

(III)

wobei die Symbole und Indizes folgende Bedeutungen haben:

| | |
|---|---|
| n' | ist eine natürliche Zahl von 0 bis 5, vorzugsweise 0 bis 3; |
| T | ist eine ($C_1$ oder $C_2$)-Alkandiylkette, die unsubstituiert oder mit einem oder zwei ($C_1$-$C_4$)Alkylresten oder mit [($C_1$-$C_3$)-Alkoxy]-carbonyl substituiert ist; |
| W | ist ein unsubstituierter oder substituierter divalenter heterocyclischer Rest aus der Gruppe der teilungesättigten oder aromatischen Fünfring-Heterocyclen mit 1 bis 3 Heteroringatomen des Typs N oder O, wobei mindestens ein N-Atom und höchstens ein O-Atom im Ring enthalten ist, vorzugsweise ein Rest aus der Gruppe (W1) bis (W4), |

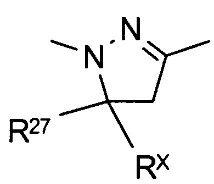

(W1)

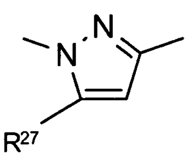

(W2)

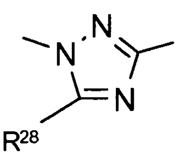

(W3)

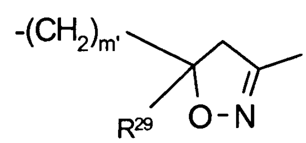

(W4)

| | |
|---|---|
| m' | ist 0 oder 1; |
| $R^{17}$, $R^{19}$ | sind gleich oder verschieden Halogen, ($C_1$-$C_4$)Alkyl, ($C_1$-$C_4$)Alkoxy, Nitro oder ($C_1$-$C_4$)Haloalkyl, |
| $R^{18}$, $R^{20}$ | sind gleich oder verschieden $OR^{24}$, $SR^{24}$ oder $NR^{24}R^{25}$ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (II) bzw. (III) verbunden ist und unsubstituiert oder durch Reste aus der Gruppe ($C_1$-$C_4$)Alkyl, ($C_1$-$C_4$)Alkoxy oder gegebenenfalls substituiertes Phenyl substituiert ist, vorzugsweise ein Rest der Formel $OR^{24}$, $NHR^{25}$ oder $N(CH_3)_2$, insbesondere der Formel $OR^{24}$; |
| $R^{24}$ | ist Wasserstoff oder ein unsubstituierter oder substituierter aliphatischer Kohlenwasserstoffrest, vorzugsweise mit insgesamt 1 bis 18 C-Atomen; |
| $R^{25}$ | ist Wasserstoff, ($C_1$-$C_6$)Alkyl, ($C_1$-$C_6$)Alkoxy oder substituiertes oder unsubstituiertes Phenyl; |
| $R^X$ | ist H, ($C_1$-$C_8$)Alkyl, $C_1$-$C_8$(Haloalkyl), ($C_1$-$C_4$)Alkoxy($C_1$-$C_8$)Alkyl, Cyano oder $COOR^{26}$, worin $R^{26}$ Wasserstoff, ($C_1$-$C_8$)Alkyl, ($C_1$-$C_8$)Haloalkyl, ($C_1$-$C_4$)Alkoxy- ($C_1$-$C_4$)alkyl, ($C_1$-$C_6$)Hydroxyalkyl, ($C_3$-$C_{12}$)Cycloalkyl oder Tri-($C_1$-$C_4$)-alkyl- silyl ist; |
| $R^{27}$, $R^{28}$, $R^{29}$ | sind gleich oder verschieden Wasserstoff, ($C_1$-$C_8$)Alkyl, ($C_1$-$C_8$)Haloalkyl, ($C_3$-$C_{12}$)Cycloalkyl oder |

substituiertes oder unsubstituiertes Phenyl;

$R^{21}$ ist $(C_1-C_4)$Alkyl, $(C_1-C_4)$Haloalkyl, $(C_2-C_4)$Alkenyl, $(C_2-C_4)$Haloalkenyl, $(C_3-C_7)$Cycloalkyl, vorzugsweise Dichlormethyl;

$R^{22}$, $R^{23}$ ist gleich oder verschieden Wasserstoff, $(C_1-C_4)$Alkyl, $(C_2-C_4)$Alkenyl, $(C_2-C_4)$Alkinyl, $(C_1-C_4)$Haloalkyl, $(C_2-C_4)$Haloalkenyl, $(C_1-C_4)$Alkylcarbamoyl- $(C_1-C_4)$alkyl, $(C_2-C_4)$Alkenylcarbamoyl-$(C_1-C_4)$alkyl, $(C_1-C_4)$Alkoxy-$(C_1- C_4)$alkyl, Dioxolanyl-$(C_1-C_4)$alkyl, Thiazolyl, Furyl, Furylalkyl, Thienyl, Piperidyl, substituiertes oder unsubstituiertes Phenyl, oder $R^{22}$ und $R^{23}$ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;

b) eine oder mehreren Verbindungen aus Gruppe:

1,8-Naphthalsäureanhydrid,
Methyl-diphenylmethoxyacetat,
1-(2-Chlorbenzyl)-3-(1-methyl-1-phenylethyl)harnstoff (Cumyluron), O,O-Diethyl S-2-ethylthioethyl phosphordithioat (Disulfoton),
4-Chlorphenyl-methylcarbamat (Mephenate),
O,O-Diethyl-O-phenylphosphorotioat (Dietholate),
4-Carboxy-3,4-dihydro-2H-1-benzopyran-4-essigsäure (CL-304415, CAS-Regno: 31541-57-8),
Cyanomethoxyimino(phenyl)acetonitril (Cyometrinil),
1,3-Dioxolan-2-ylmethoxyimino(phenyl)acetonitril (Oxabetrinil), 4'-Chlor-2,2,2-trifluoracetophenon-O-1,3-dioxolan-2-ylmethyloxim (Fluxofenim), 4,6-Dichlor-2-phenylpyrimidin (Fenclorim),
Benzyl-2-chlor-4-trifluormethyl-1,3-thiazol-5-carboxylat (Flurazole), 2-Dichlormethyl-2-methyl-1,3-dioxolan (MG-191),
N-(4-Methylphenyl)-N'-(1-methyl-1-phenylethyl)harnstoff (Dymron), (2,4-Dichlorphenoxy)essigsäure (2,4-D),
(4-Chlorphenoxy)essigsäure,
(R,S)-2-(4-Chlor-o-tolyloxy)propionsäure (Mecoprop),
4-(2,4-Dichlorphenoxy)buttersäure (2,4-DB),
(4-Chlor-o-tolyloxy)essigsäure (MCPA),
4-(4-Chlor-o-tolyloxy)buttersäure,
4-(4-Chlorphenoxy)buttersäure,
3,6-Dichlor-2-methoxybenzoesäure (Dicamba),
1-(Ethoxycarbonyl)ethyl 3,6-dichlor-2-methoxybenzoat (Lactidichlor) sowie deren Salze und Ester, vorzugsweise $(C_1-C_8)$;

c) N-Acylsulfonamide der Formel (IV) und ihre Salze,

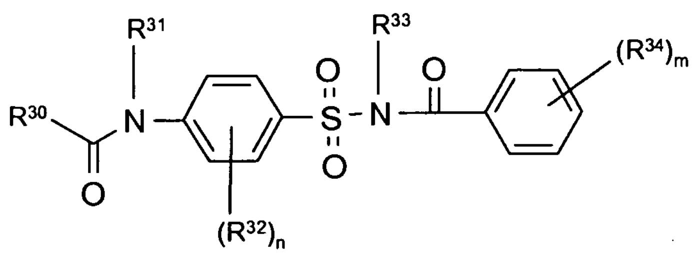

(IV)

worin

$R^{30}$ Wasserstoff, einen Kohlenwasserstoffrest, einen Kohlenwasserstoffoxyrest, einen Kohlenwasserstoffthiorest oder einen Heterocyclylrest, der vorzugsweise über ein C-Atom gebunden ist, wobei jeder der letztgenannten

4 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel $-Z^a-R^a$ substituiert ist, wobei jeder Kohlenwasserstoffteil vorzugsweise 1 bis 20 C-Atome aufweist und ein C-haltiger Rest $R^{30}$ inklusive Substituenten vorzugsweise 1 bis 30 C-Atome auf-

weist;

| | |
|---|---|
| $R^{31}$ | Wasserstoff oder $(C_1-C_4)$-Alkyl, vorzugsweise Wasserstoff, oder |
| $R^{30}$ und $R^{31}$ | zusammen mit der Gruppe der Formel -CO-N- den Rest eines 3- bis 8-gliedrigen gesättigten oder ungesättigten Rings; |
| $R^{32}$ | gleich oder verschieden Halogen, Cyano, Nitro, Amino, Hydroxy, Carboxy, Formyl, $CONH_2$, $SO_2NH_2$ oder einen Rest der Formel $-Z^b-R^b$; |
| $R^{33}$ | Wasserstoff oder $(C_1-C_4)$-Alkyl, vorzugsweise H; |
| $R^{34}$ | gleich oder verschieden Halogen, Cyano, Nitro, Amino, Hydroxy, Carboxy, CHO, $CONH_2$, $SO_2NH_2$ oder einen Rest der Formel $-Z^c-R^c$; |
| $R^a$ | einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[$(C_1-C_4)$-alkyl]-amino substituiert ist, oder einen Alkylrest, in dem mehrere, vorzugsweise 2 oder 3, nicht benachbarte $CH_2$-Gruppen jeweils durch ein Sauerstoffatom ersetzt sind; |
| $R^b$,$R^c$ | gleich oder verschieden einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen-$(C_1-C_4)$-alkoxy, Mono- und Di-[$(C_1-C_4)$-alkyl]-amino substituiert ist, oder einen Alkylrest, in dem mehrere, vorzugsweise 2 oder 3, nicht benachbarte $CH_2$-Gruppen jeweils durch ein Sauerstoffatom ersetzt sind; |
| $Z^a$ | eine divalente Gruppe der Formel -O-, -S-, -CO-, -CS-, -CO-O-, -CO-S-, -O-CO-, -S-CO-, -SO-, $-SO_2-$, -NR*-, -CO-NR*-, -NR*-CO-, $-SO_2$-NR*- oder -NR*-$SO_2$-, wobei die rechts angegebene Bindung der jeweiligen divalenten Gruppe die Bindung zum Rest $R^a$ ist und wobei die R* in den letztgenannten 5 Resten unabhängig voneinander jeweils H, $(C_1-C_4)$-Alkyl oder Halo-$(C_1-C_4)$- alkyl bedeuten; |
| $Z^b$,$Z^c$ | unabhängig voneinander eine direkte Bindung oder eine divalente Gruppe der Formel -O-, -S-, -CO-, -CS-, -CO-O-, -CO-S-, -O-CO-, -S-CO-, -SO-, $-SO_2-$, -NR*-, $-SO_2$-NR*-, -NR*-$SO_2$-, -CO-NR*- oder -NR*-CO-, wobei die rechts angegebene Bindung der jeweiligen divalenten Gruppe die Bindung zum Rest $R^b$ bzw. $R^c$ ist und wobei die R* in den letztgenannten 5 Resten unabhängig voneinander jeweils H, $(C_1-C_4)$-Alkyl oder Halo-$(C_1-C_4)$-alkyl bedeuten; |
| n | eine ganze Zahl von 0 bis 4, vorzugsweise 0, 1 oder 2, insbesondere 0 oder 1, und |
| m | eine ganze Zahl von 0 bis 5, vorzugsweise 0, 1, 2 oder 3, insbesondere 0, 1 oder 2; bedeuten; |

d) Acylsulfamoylbenzoesäureamide der allgemeinen Formel (V), gegebenenfalls auch in Salzform,

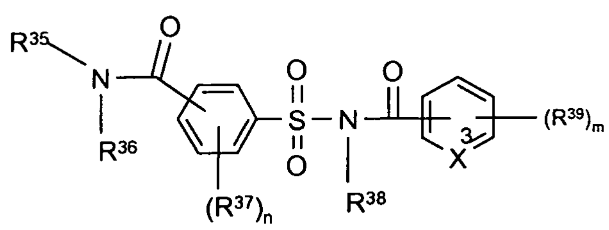

(V)

worin

| | |
|---|---|
| $X^3$ | CH oder N; |
| $R^{35}$ | Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, $CONH_2$, $SO_2NH_2$ und $Z^a-R^a$ substituiert sind; |
| $R^{36}$ | Wasserstoff, Hydroxy, $(C_1-C_6)$-Alkyl, $(C_2-C_6)$-Alkenyl, $(C_2-C_6)$-Alkinyl, $(C_1-C_6)$- Alkoxy, $(C_2-C_6)$-Alkenyloxy, wobei die fünf letztgenannten Reste gegebenenfalls durch einen oder mehrere, gleiche oder verschiedene Reste aus der Gruppe Halogen, Hydroxy, $(C_1-C_4)$-Alkyl, $(C_1-C_4)$-Alkoxy und $(C_1-C_4)$-Alkylthio substituiert sind, oder |
| $R^{35}$ und $R^{36}$ | zusammen mit dem sie tragenden Stickstoffatom einen 3- bis 8- gliedrigen gesättigten oder ungesättigten Ring; |
| $R^{37}$ | Halogen, Cyano, Nitro, Amino, Hydroxy, Carboxy, CHO, $CONH_2$, $SO_2NH_2$ oder $Z^b-R^b$; |

$R^{38}$ Wasserstoff, $(C_1-C_4)$-Alkyl, $(C_2-C_4)$-Alkenyl oder $(C_2-C_4)$-Alkinyl;

$R^{39}$ Halogen, Cyano, Nitro, Amino, Hydroxy, Carboxy, Phosphoryl, CHO, $CONH_2$, $SO_2NH_2$ oder $Z^c-R^c$,

$R^a$ einen $(C_2-C_{20})$-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[$(C_1-C_4)$-alkyl]-amino substituiert sind;

$R^b$,$R^c$ gleich oder verschieden einen $(C_2-C_{20})$-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, $(C_1-C_4)$- Haloalkoxy, Mono- und Di-[$(C_1-C_4)$-alkyl]-amino substituiert sind;

$Z^a$ eine divalente Einheit aus der Gruppe O, S, CO, CS, C(O)O, C(O)S, SO, $SO_2$, $NR^d$, $C(O)NR^d$ oder $SO_2NR^d$;

$Z^b$, $Z^c$ gleich oder verschieden eine direkte Bindung oder eine divalente Einheit aus der Gruppe O, S, CO, CS, C(O)O, C(O)S, SO, $SO_2$, $NR^d$, $SO_2NR^d$ oder $C(O)NR^d$;

$R^d$ Wasserstoff, $(C_1-C_4)$-Alkyl oder $(C_1-C_4)$-Haloalkyl;

n eine ganze Zahl von 0 bis 4, und

m für den Fall, dass X für CH steht, eine ganze Zahl von 0 bis 5, und für den Fall, dass X für N steht, eine ganze Zahl von 0 bis 4 bedeuten;

e) Verbindungen von Typ der Acylsulfamoylbenzoesäureamide, z.B. der nachfolgenden Formel (VI), die z.B. bekannt sind aus WO 99/16744,

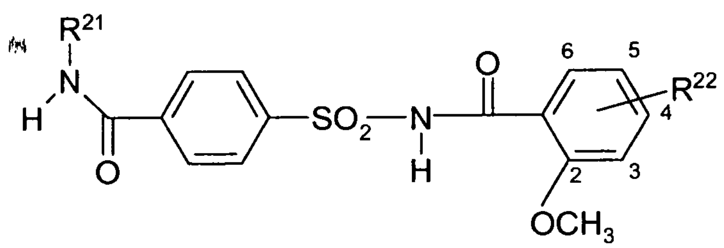

**(VI)**

z.B. solche worin

$R^{21}$ = Cyclo-Propyl und $R^{22}$ = H ist (S3-1 = 4-Cyclopopylaminocarbonyl-N-(2 methoxybenzoyl)benzolsulfonamid),

$R^{21}$ = Cyclo-Propyl und $R^{22}$ = 5-Cl ist (S3-2),

$R^{21}$ = Ethyl und $R^{22}$ = H ist (S3-3),

$R^{21}$ = iso-Propyl und $R^{22}$ = 5-Cl ist (S3-4) und

$R^{21}$ = iso-Propyl und $R^{22}$ = H ist (S3-5);

f) Verbindungen vom Typ der N-Acylsulfamoylphenylharnstoffe der Formel (VII), die z.B. bekannt sind aus der EP-A-365484,

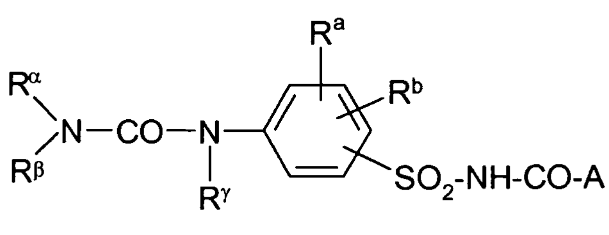

**(VII)**

worin

A für einen Rest aus der Gruppe

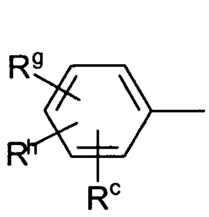,
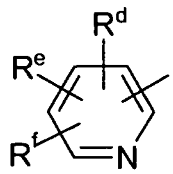,
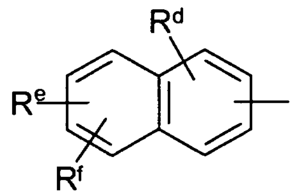,

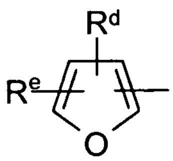 oder 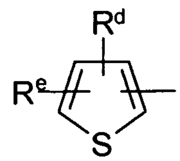

steht,

$R^{\alpha}$ und $R^{\beta}$ unabhängig voneinander für Wasserstoff, $C_1$-$C_8$-Alkyl, $C_3$-$C_8$-Cycloalkyl, $C_3$-$C_6$ Alkenyl, $C_3$-$C_6$-Alkinyl,

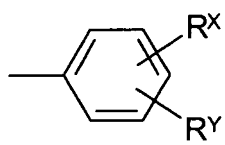

oder durch $C_1$-$C_4$-Alkoxyoder

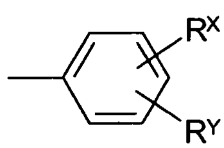

substituiertes $C_1$-$C_4$-Alkoxy stehen, oder

$R^{\alpha}$ und $R^{\beta}$ gemeinsam für eine $C_4$-$C_6$-Alkylenbrücke oder eine durch Sauerstoff, Schwefel, SO, $SO_2$, NH oder -N ($C_1$-$C_4$-Alkyl)- unterbrochene $C_4$-$C_6$-Alkylenbrücke stehen,

$R^{\gamma}$ für Wasserstoff oder $C_1$-$C_4$-Alkyl steht,

$R^a$ und $R^b$ unabhängig voneinander für Wasserstoff, Halogen, Cyan, Nitro, Trifluormethyl, $C_1$-$C_4$-Alkyl, $C_1$-$C_4$-Alkoxy, $C_1$-$C_4$-Alkylthio, $C_1$-$C_4$- Alkylsulfinyl, $C_1$-$C_4$-Alkylsulfonyl, $-COOR^j$, $-CONR^kR^m$, $-COR^n$, $-SO_2NR^kR^m$ oder $-OSO_2$-$C_1$-$C_4$-Alkyl , oder $R^a$ und $R^b$ gemeinsam für eine $C_3$-$C_4$- Alkylenbrücke, die durch Halogen oder $C_1$-$C_4$-Alkyl substituiert sein kann, oder eine $C_3$-$C_4$-Alkenylenbrücke, die durch Halogen oder $C_1$-$C_4$-Alkyl substituiert sein kann, oder eine $C_4$-Alkadienylenbrücke, die durch Halogen oder $C_1$-$C_4$-Alkyl substituiert sein kann, und

$R^g$ und $R^h$ unabhängig voneinander für Wasserstoff, Halogen, $C_1$-$C_4$-Alkyl, Trifluormethyl, Methoxy, Methylthio oder $-COOR^j$ stehen, wobei

$R^c$ für Wasserstoff, Halogen, $C_1$-$C_4$-Alkyl oder Methoxy steht,

$R^d$ für Wasserstoff, Halogen, Nitro, $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $-COOR^j$ oder $-CONR^kR^m$ steht,

$R^e$ für Wasserstoff, Halogen, $C_1$-$C_4$-Alkyl, $-COOR^j$, Trifluormethyl oder Methoxy steht, oder $R^d$ und $R^e$ gemeinsam für eine $C_3$-$C_4$-Alkylenbrücke stehen,

| | |
|---|---|
| $R^f$ | für Wasserstoff, Halogen oder $C_1$-$C_4$-Alkyl steht, |
| $R^X$ und $R^Y$ | unabhängig voneinander für Wasserstoff, Halogen, $C_1$-$C_4$-Alkyl, $C_1$-$C_4$- Alkoxy, $C_1$-$C_4$-Alkylthio, -$COOR^j$, Trifluormethyl, Nitro oder Cyan stehen, |
| $R^j$, $R^k$ und $R^m$ | unabhängig voneinander für Wasserstoff oder $C_1$-$C_4$-Alkyl stehen, |
| $R^k$ und $R^m$ | gemeinsam für eine $C_4$-$C_6$-Alkylenbrücke oder eine durch Sauerstoff, NH oder -N($C_1$-$C_4$-Alkyl)-unterbrochene $C_4$-$C_6$-Alkylenbrücke stehen, und |
| $R^n$ | $C_1$-$C_4$-Alkyl, Phenyl oder durch Halogen, $C_1$-$C_4$-Alkyl, Methoxy, Nitro oder Trifluormethyl substituiertes Phenyl bedeutet, |

bevorzugt
1-[4-(N-2-Methoxybenzoylsulfamoyl)phenyl]-3-methylharnstoff,
1-[4-(N-2-Methoxybenzoylsulfamoyl)phenyl]-3,3-dimethylharnstoff,
1-[4-(N-4,5-Dimethylbenzoylsulfamoyl)phenyl]-3-methylharnstoff,
1-[4-(N-Naphthoylsulfamoyl)phenyl]-3,3-dimethylharnstoff,

g) Verbindungen vom Typ der Acylsulfamoylbenzoesäureamide der Formel (VIII), bekannt aus EP-A-1019368, gegebenenfalls auch in Salzform,

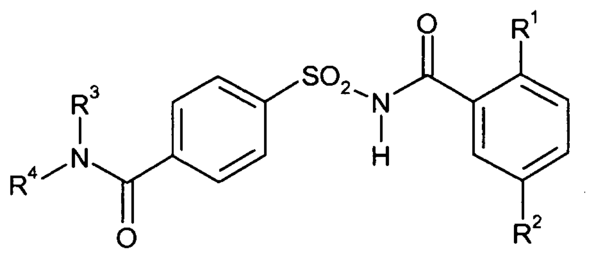

**(VIII)**

worin

| | |
|---|---|
| $R^1$ | bedeutet Methyl, Methoxy oder Trifluormethoxy; |
| $R^2$ | bedeutet Wasserstoff, Chlor oder Methyl; |
| $R^3$ | bedeutet Wasserstoff, Ethyl oder Propargyl; |
| $R^4$ | bedeutet Ethyl, Cyclopropyl, iso-Propyl oder Propargyl, oder |
| $R^3$ und $R^4$ | bilden gemeinsam die Gruppe $(CH_2)_4$, |

einschließlich der Stereoisomeren und der in der Landwirtschaft gebräuchlichen Salze.

Die Verbindungen der Formel (I) sind z.B. aus EP-A-0 333 131 (ZA-89/1960), EP-A-0 269 806 (US-A-4,891,057), EP-A-0 346 620 (AU-A-89/34951), EP-A-0 174 562, EP-A-0 346 620 (WO-A-91/08 202), WO-A-91/07 874 oder WO-A 95/07 897 (ZA 94/7120) und der dort zitierten Literatur bekannt oder können nach oder analog den dort beschriebenen Verfahren hergestellt werden.

Die Verbindungen der Formel (II) sind aus EP-A-0 086 750, EP-A-0 94349 (US-A-4,902,340), EP-A-0 191736 (US-A-4,881,966) und EP-A-0 492 366 und dort zitierter Literatur bekannt oder können nach oder analog den dort beschriebenen Verfahren hergestellt werden. Einige Verbindungen sind ferner in EP-A-0 582 198 und WO 2002/34048 beschrieben.

Die Verbindungen der Formel (III) sind aus zahlreichen Patentanmeldungen bekannt, beispielsweise US-A-4,021,224 und US-A-4,021,229.

Verbindungen der Gruppe (b) sind weiterhin aus CN-A- 87/102 789, EP-A-365484 sowie aus "The Pesticide Manual", The British Crop Protection Council and the Royal Society of Chemistry, 11th edition, Farnham 1997, bekannt.

Die Verbindungen der Gruppe (c) sind in der WO-A-97/45016, die der Gruppe (d) in der WO-A-99/16744, die der Gruppe B (e) in der EP-A-365484 und die der Gruppe (g) in EP-A-1019368 beschrieben.

Die zitierten Schriften enthalten ausführliche Angaben zu Herstellungsverfahren und Ausgangsmaterialien und nennen bevorzugte Verbindungen. Auf diese Schriften wird ausdrücklich Bezug genommen, sie gelten durch Zitat als Bestandteil dieser Beschreibung.

Bevorzugt sind als Safener bekannte Verbindungen der Formel (I) und/oder (II) bei denen die Symbole und

Indizes folgende Bedeutungen haben:

$R^{24}$ ist Wasserstoff, ($C_1$-$C_{18}$)-Alkyl, ($C_3$-$C_{12}$)-Cycloalkyl, ($C_2$-$C_8$)-Alkenyl und ($C_2$-$C_{18}$)Alkinyl, wobei die C-haltigen Gruppen durch einen oder mehrere, vorzugsweise bis zu drei, Reste $R^{50}$ substituiert sein können;

$R^{50}$ ist gleich oder verschieden Halogen, Hydroxy, ($C_1$-$C_8$)-Alkoxy, ($C_1$-$C_8$)Alkylthio, ($C_2$-$C_8$)Alkenylthio, ($C_2$-$C_8$) Alkinylthio, ($C_2$-$C_8$)Alkenyloxy, ($C_2$-$C_8$)Alkinyloxy, ($C_3$-$C_7$)Cycloalkyl, ($C_3$-$C_7$)Cycloalkoxy, Cyano, Mono- und Di-($C_1$-$C_4$)-alkyl)-amino, Carboxy, ($C_1$-$C_8$)Alkoxycarbonyl, ($C_2$-$C_8$)Alkenyloxycarbonyl, ($C_1$-$C_8$)Alkylthiocarbonyl, ($C_2$-$C_8$)Alkinyloxycarbonyl, ($C_1$-$C_8$)Alkylcarbonyl, ($C_2$-$C_8$)Alkenylcarbonyl, ($C_2$-$C_8$)Alkinylcarbonyl, 1-(Hydroxyimino)-($C_1$-$C_6$)-alkyl, 1-[($C_1$-$C_4$)Alkylimino]- ($C_1$-$C_4$)-alkyl, 1-[($C_1$-$C_4$)Alkoxyimino]-($C_1$-$C_6$)-alkyl, ($C_1$-$C_8$) Alkylcarbonylamino, ($C_2$-$C_8$)Alkenylcarbonylamino, ($C_2$-$C_8$)Alkinylcarbonylamino, Aminocarbonyl, ($C_1$-$C_8$)Alkylaminocarbonyl, Di-$C_1$-$C_6$)-alkylaminocarbonyl, ($C_2$-$C_6$)Alkenylaminocarbonyl, ($C_2$-$C_6$)Alkinylaminocarbonyl, ($C_1$-$C_8$)Alkoxycarbonylamino, ($C_1$-$C_8$)Alkylaminocarbonylamino, ($C_1$-$C_6$)Alkylcarbonyloxy, das unsubstituiert oder durch $R^{51}$ substituiert ist, ($C_2$-$C_6$)Alkenylcarbonyloxy, ($C_2$-$C_6$)Alkinylcarbonyloxy, ($C_1$-$C_8$)Alkylsulfonyl, Phenyl, Phenyl-($C_1$-$C_6$)-alkoxy, Phenyl-($C_1$-$C_6$)-alkoxycarbonyl, Phenoxy, Phenoxy-($C_1$-$C_6$)-alkoxy, Phenoxy-($C_1$-$C_6$)-alkoxycarbonyl, Phenylcarbonyloxy, Phenylcarbonylamino, Phenyl-($C_1$-$C_6$)- alkylcarbonylamino, wobei die letztgenannten 9 Reste im Phenylring unsubstituiert oder ein- oder mehrfach, vorzugsweise bis zu dreifach durch Reste $R^{52}$ substituiert sind; $SiR'_3$, $-O-SiR'_3$, $R'_3Si-(C_1-C_8)$-alkoxy, $-CO-O-NR'_2$, - $O-N=CR'_2$, $-N=CR'_2$, $-O-NR'_2$, $-NR'_2$, $CH(OR')_2$, $-O-(CH_2)_m-CH(OR')_2$, - $CR'''(OR')_2$, $-O-(CH_2)_mCR'''(OR'')_2$ oder durch R"O-CHR"'CHCOR"-($C_1$-$C_6$)- alkoxy,

$R^{51}$ ist gleich oder verschieden Halogen, Nitro, ($C_1$-$C_4$)Alkoxy und unsubstituiertes oder mit einem oder mehreren, vorzugsweise bis zu drei Resten $R^{52}$ substituiertes Phenyl;

$R^{52}$ ist gleich oder verschieden Halogen, ($C_1$-$C_4$)Alkyl, ($C_1$-$C_4$)Alkoxy, ($C_1$-$C_4$)Haloalkyl, ($C_1$-$C_4$)-Haloalkoxy oder Nitro;

R' ist gleich oder verschieden Wasserstoff, ($C_1$-$C_4$)Alkyl, unsubstituiertes oder durch einen oder mehrere, vorzugsweise bis zu drei, Reste $R^{52}$ substituiertes Phenyl oder zwei Reste R' bilden zusammen eine ($C_2$-$C_6$) Alkandiylkette;

R" ist gleich oder verschieden ($C_1$-$C_4$)Alkyl oder zwei Reste R" bilden zusammen eine ($C_2$-$C_6$)Alkandiylkette;

R"' ist Wasserstoff oder ($C_1$-$C_4$)Alkyl;

m ist 0, 1, 2, 3, 4, 5 oder 6.

Besonders bevorzugt sind als Safener bekannte Verbindungen der Formel (I) und/oder (II), bei denen die Symbole und Indizes folgende Bedeutungen haben:

$R^{24}$ ist Wasserstoff, ($C_1$-$C_8$)Alkyl oder ($C_3$-$C_7$)Cycloalkyl, wobei die vorstehenden C-haltigen Reste unsubstituiert sind oder ein- oder mehrfach durch Halogen oder ein- oder zweifach, vorzugsweise einfach, durch Reste $R^{50}$ substituiert sind,

$R^{50}$ ist gleich oder verschieden Hydroxy, ($C_1$-$C_4$)Alkoxy, Carboxy, ($C_1$-$C_4$)Alkoxycarbonyl, ($C_2$-$C_6$)Alkenyloxycarbonyl, ($C_2$-$C_6$)Alkinyloxycarbonyl, 1-(Hydroxyimino)-($C_1$-$C_4$)-alkyl, 1-[($C_1$-$C_4$)Alkylimino]-($C_1$-$C_4$)-alkyl und 1-[($C_1$-$C_4$)Alkoxyimino]-($C_1$-$C_4$)-alkyl; $-SiR'_3$, $-O-N=CR'_2$, $-N=CR'_2$, $-NR'_2$, und $-O-NR'_2$, worin R' gleich oder verschieden Wasserstoff, ($C_1$-$C_4$)Alkyl oder paarweise eine ($C_4$-$C_5$)Alkandiylkette bedeutet,

$R^{27}$, $R^{28}$, $R^{29}$ sind gleich oder verschieden Wasserstoff, ($C_1$-$C_8$)Alkyl, ($C_1$-$C_6$)Haloalkyl, ($C_3$-$C_7$)Cycloalkyl oder Phenyl, das unsubstituiert oder durch einen oder mehrere der Reste aus der Gruppe Halogen, Cyano, Nitro, Amino, Mono- und Di-[($C_1$-$C_4$)alkyl]-amino, ($C_1$-$C_4$)Alkyl, ($C_1$-$C_4$)Haloalkyl, ($C_1$- $C_4$)Alkoxy, ($C_1$-$C_4$)Haloalkoxy, ($C_1$-$C_4$)Alkylthio und ($C_1$-$C_4$)Alkylsulfonyl substituiert ist;

$R^x$ ist Wasserstoff oder $COOR^{26}$, worin $R^{26}$ Wasserstoff, ($C_1$-$C_8$)Alkyl, ($C_1$- $C_8$)Haloalkyl, ($C_1$-$C_4$-Alkoxy)-($C_1$-$C_4$)-alkyl, ($C_1$-$C_6$)Hydroxyalkyl, ($C_3$- $C_7$)Cycloalkyl oder Tri-($C_1$-$C_4$)-alkylsilyl bedeutet,

$R^{17}$, $R^{19}$ sind gleich oder verschieden Halogen, Methyl, Ethyl, Methoxy, Ethoxy, ($C_1$ oder $C_2$)-Haloalkyl, vorzugsweise Wasserstoff, Halogen oder ($C_1$ oder $C_2$)- Haloalkyl.

Ganz besonders bevorzugt sind als Safener bekannte Verbindungen in welchen die Symbole und Indizes in Formel (I) folgende Bedeutungen haben:

$R^{17}$ ist Halogen, Nitro oder ($C_1$-$C_4$)Haloalkyl,

n' ist 0, 1, 2 oder 3;

$R^{18}$ ist ein Rest der Formel $OR^{24}$,

$R^{24}$ ist Wasserstoff, ($C_1$-$C_8$)Alkyl oder ($C_3$-$C_7$)Cycloalkyl, wobei die vorstehenden C-haltigen Reste unsubstituiert sind oder ein- oder mehrfach, vorzugsweise bis zu dreifach, durch gleiche oder verschiedene

Halogen-Reste oder bis zu zweifach, vorzugsweise einfach, durch gleiche oder verschiedene Reste aus der Gruppe Hydroxy, $(C_1-C_4)$Alkoxy, $(C_1-C_4)$Alkoxycarbonyl, $(C_2-C_6)$Alkenyloxycarbonyl, $(C_2-C_6)$Alkinyloxycarbonyl, 1-(Hydroxyimino)-$(C_1-C_4)$- alkyl, 1-[$(C_1-C_4)$Alkylimino]-$(C_1-C_4)$-alkyl, 1-[$(C_1-C_4)$Alkoxyimino]-$(C_1-C_4)$-alkyl und Reste der Formeln $-SiR'_3$, $-O-N=R'_2$, $-N=CR'_2$, $-NR'_2$ und $-O-NR'_2$ substituiert sind, wobei die Reste R' in den genannten Formeln gleich oder verschieden Wasserstoff, $(C_1-C_4)$Alkyl oder paarweise ($C_4$ oder $C_5$)Alkandiyl bedeuten;

$R^{27}$, $R^{28}$, $R^{29}$ sind gleich oder verschieden Wasserstoff, $(C_1-C_8)$Alkyl, $(C_1-C_6)$Haloalkyl, $(C_3-C_7)$Cycloalkyl oder Phenyl, das unsubstituiert oder durch einen oder mehrere der Reste aus der Gruppe Halogen, $(C_1-C_4)$Alkyl, $(C_1-C_4)$Alkoxy, Nitro, $(C_1-C_4)$Haloalkyl und $(C_1-C_4)$Haloalkoxy substituiert ist, und

$R^x$ ist Wasserstoff oder $COOR^{26}$, worin $R^{26}$ Wasserstoff, $(C_1-C_8)$Alkyl, $(C_1-C_8)$Haloalkyl, $(C_1-C_4)$Alkoxy-$(C_1-C_4)$-alkyl, $(C_1-C_6)$Hydroxyalkyl, $(C_3-C_7)$Cycloalkyl oder Tri-$(C_1-C_4)$-alkylsilyl ist.

Ganz besonders bevorzugt sind auch als Safener bekannte Verbindungen der Formel (II), bei denen die Symbole und Indizes folgende Bedeutungen haben:

$R^{19}$ ist Halogen oder $(C_1-C_4)$Haloalkyl;

n' ist 0, 1, 2 oder 3, wobei $(R^{19})_{n'}$ vorzugsweise 5-Cl ist;

$R^{20}$ ist ein Rest der Formel $OR^{24}$;

T ist $CH_2$ oder CH(COO-$(C_1-C_3$-Alkyl)) und

$R^{24}$ ist Wasserstoff, $(C_1-C_8)$Alkyl, $(C_1-C_8)$Haloalkyl oder $(C_1-C_4)$Alkoxy-$(C_1-C_4)$- alkyl, vorzugsweise Wasserstoff oder $(C_1-C_8)$Alkyl.

Insbesondere bevorzugt sind dabei als Safener bekannte Verbindungen der Formel (I) bei denen die Symbole und Indizes folgende Bedeutungen haben:

W ist (W1);

$R^{17}$ ist Halogen oder $(C_1-C_2)$Haloalkyl;

n' ist 0, 1, 2 oder 3, wobei $(R^{17})_{n'}$ vorzugsweise 2,4-$Cl_2$ ist;

$R^{1a}$ ist ein Rest der Formel $OR^{24}$;

$R^{24}$ ist Wasserstoff, $(C_1-C_8)$Alkyl, $(C_1-C_4)$Haloalkyl, $(C_1-C_4)$Hydroxyalkyl, $(C_3-C_7)$Cycloalkyl, $(C_1-C_4)$Alkoxy-$(C_1-C_4)$-alkyl oder Tri-$(C_1-C_2)$-alkylsilyl, vorzugsweise $(C_1-C_4)$Alkyl,

$R^{27}$ ist Wasserstoff, $(C_1-C_8)$Alkyl, $(C_1-C_4)$Haloalkyl oder $(C_3-C_7)$Cycloalkyl, vorzugsweise Wasserstoff oder $(C_1-C_4)$Alkyl, und

$R^x$ ist $COOR^{26}$, worin $R^{26}$ Wasserstoff, $(C_1-C_8)$Alkyl, $(C_1-C_4)$Haloalkyl, $(C_1-C_4)$Hydroxyalkyl, $(C_3-C_7)$Cycloalkyl, $(C_1-C_4)$Alkoxy-$(C_1-C_4)$-alkyl oder Tri-$(C_1-C_2)$-alkylsilyl, vorzugsweise Wasserstoff oder $(C_1-C_4)$Alkyl ist.

Insbesondere bevorzugt sind auch als Safener bekannte Verbindungen der Formel (I), worin die Symbole und Indizes folgende Bedeutungen haben:

W ist (W2);

$R^{17}$ ist Halogen oder $(C_1-C_2)$Haloalkyl;

n' ist 0, 1, 2 oder 3, wobei $(R^{17})_{n'}$ vorzugsweise 2,4-$Cl_2$ ist;

$R^{1a}$ ist ein Rest der Formel $OR^{24}$;

$R^{24}$ ist Wasserstoff, $(C_1-C_8)$Alkyl, $(C_1-C_4)$Haloalkyl, $(C_1-C_4)$Hydroxyalkyl, $(C_3-C_7)$Cycloalkyl, $(C_1-C_4$-Alkoxy)-$C_1-C_4$-alkyl oder Tri-$(C_1-C_2)$-alkyl-silyl, vorzugsweise $(C_1-C_4)$Alkyl, und

$R^{27}$ ist Wasserstoff, $(C_1-C_8)$Alkyl, $(C_1-C_4)$Haloalkyl, $(C_3-C_7)$Cycloalkyl oder unsubstituiertes oder substituiertes Phenyl, vorzugsweise Wasserstoff, $(C_1-C_4)$Alkyl oder Phenyl, das unsubstituiert oder durch einen oder mehrere Reste aus der Gruppe Halogen, $(C_1-C_4)$Alkyl, $(C_1-C_4)$Haloalkyl, Nitro, Cyano oder $(C_1-C_4)$Alkoxy substituiert ist.

Insbesondere bevorzugt sind auch als Safener bekannte Verbindungen der Formel (I), worin die Symbole und Indizes folgende Bedeutungen haben:

W ist (W3);

$R^{17}$ ist Halogen oder $(C_1-C_2)$Haloalkyl;

n' ist 0, 1, 2 oder 3, wobei $(R^{17})_{n'}$ vorzugsweise 2,4-$Cl_2$ ist;

$R^{18}$ ist ein Rest der Formel $OR^{24}$;

$R^{24}$ ist Wasserstoff, $(C_1-C_8)$Alkyl, $(C_1-C_4)$Haloalkyl, $(C_1-C_4)$Hydroxyalkyl, $(C_3-C_7)$Cycloalkyl, $(C_1-C_4)$Alkoxy-$(C_1-C_4)$-alkyl oder Tri-$(C_1-C_2)$-alkylsilyl, vorzugsweise $(C_1-C_4)$Alkyl, und

$R^{28}$ ist $(C_1-C_8)$Alkyl oder $(C_1-C_4)$Haloalkyl, vorzugsweise $C_1$-Haloalkyl.

Insbesondere bevorzugt sind auch als Safener bekannte Verbindungen der Formel (I), worin die Symbole und Indizes folgende Bedeutung haben:

W ist (W4);
$R^{17}$ ist Halogen, Nitro, $(C_1-C_4)$Alkyl, $(C_1-C_2)$Haloalkyl, vorzugsweise $CF_3$, oder $(C_1-C_4)$Alkoxy;
n' ist 0, 1, 2 oder 3;
m' ist 0 oder 1;
$R^{18}$ ist ein Rest der Formel $OR^{24}$;
$R^{24}$ ist Wasserstoff, $(C_1-C_4)$Alkyl, Carboxy-$(C_1-C_4)$-alkyl, $(C_1-C_4)$Alkoxycarbonyl- $(C_1-C_4)$-alkyl, vorzugsweise $(C_1-C_4)$Alkoxy-CO-$CH_2$-, $(C_1-C_4)$Alkoxy-CO- $C(CH_3)H$-, HO-CO-$CH_2$- oder HO-CO-$C(CH_3)H$-, und
$R^{29}$ ist Wasserstoff, $(C_1-C_4)$Alkyl, $(C_1-C_4)$Haloalkyl, $(C_3-C_7)$Cycloalkyl oder Phenyl, das unsubstituiert oder durch einen oder mehrere der Reste aus der Gruppe Halogen, $(C_1-C_4)$Alkyl, $(C_1-C_4)$Haloalkyl, Nitro, Cyano und $(C_1-C_4)$ Alkoxy substituiert ist.

Folgende Gruppen von als Safener bekannte Verbindungen sind insbesondere als Wirkstoffe für die Erhöhung der Toleranz von Pflanzen gegenüber abiotischen Stressoren geeignet:

a) Verbindungen vom Typ der Dichlorphenylpyrazolin-3-carbonsäure (d.h. der Formel (I), worin W = (W1) und $(R^{17})_{n'}$ = 2,4-$Cl_2$), vorzugsweise Verbindungen wie 1-(2,4-Dichlorphenyl)-5-(ethoxycarbonyl)-5-methyl-2-pyrazolin-3-carbonsäureethylester (I-1, Mefenpyr-diethyl), Mefenpyr-dimethyl und Mefenpyr (I-0), und verwandte Verbindungen, wie sie in der WO-A 91/07874 beschrieben sind;

b) Derivate der Dichlorphenylpyrazolcarbonsäure (d.h. der Formel (I), worin W = (W2) und $(R^{17})_{n'}$ = 2,4-$Cl_2$ ist), vorzugsweise Verbindungen wie 1-(2,4-Dichlorphenyl)-5-methyl-pyrazol-3-carbonsäureethylester (I-2), 1-(2,4-Dichlorphenyl)-5-isopropyl-pyrazol-3-carbonsäureethylester (I-3), 1-(2,4-Dichlorphenyl)-5-(1,1-dimethyl-ethyl)pyrazol-3-carbonsäureethylester (I-4), 1-(2,4-Dichlorphenyl)-5-phenyl-pyrazol-3-carbonsäureethylester (I-5) und verwandte Verbindungen, wie sie in EP-A-0 333 131 und EP-A-0 269 806 beschrieben sind;

c) Verbindungen vom Typ der Triazolcarbonsäuren (d.h. der Formel (I), worin W = (W3) und $(R^{17})_{n'}$ = 2,4-$Cl_2$ ist), vorzugsweise Verbindungen wie Fenchlorazol-ethyl, d.h. 1-(2,4-Dichlorphenyl)-5-trichlormethyl-(1H)-1,2,4-triazol-3-carbonsäureethylester (I-6), und verwandte Verbindungen (siehe EP-A-0 174 562 und EP-A-0 346 620);

d) Verbindungen vom Typ der 5-Benzyl- oder 5-Phenyl-2-isoxazolin-3-carbonsäure oder der 5,5-Diphenyl-2-isoxazolin-3-carbonsäure wie Isoxadifen (I-12), (worin W = (W4) ist), vorzugsweise Verbindungen wie 5-(2,4-Dichlorbenzyl)-2-isoxazolin-3-carbonsäureethylester (I-7) oder 5-Phenyl-2-isoxazolin-3-carbonsäureethylester (I-8) und verwandte Verbindungen, wie sie in WO-A- 91/08202 beschrieben sind, oder der 5,5-Diphenyl-2-isoxazolincarbonsäureethylester (I-9, Isoxadifen-ethyl) oder -n-propylester (I-10) oder der 5-(4-Fluorphenyl)-5-phenyl-2-isoxazolin-3-carbonsäureethylester (I-11), wie sie in der WO-A-95/07897 beschrieben sind.

e) Verbindungen vom Typ der 8-Chinolinoxyessigsäure, z.B. solche der Formel (II), worin $(R^{19})_{n'}$ = 5-Cl, $R^{20}$ = $OR^{24}$ und T = $CH_2$ ist, vorzugsweise die Verbindungen
(5-Chlor-8-chinolinoxy)essigsäure-(1-methylhexyl)-ester (II-1, Cloquintocetmexyl),
(5-Chlor-8-chinolinoxy)essigsäure-(1,3-dimethyl-but-1-yl)-ester (II-2),
(5-Chlor-8-chinolinoxy)essigsäure-4-allyl-oxy-butylester (II-3),
(5-Chlor-8-chinolinoxy)essigsäure-1-allyloxy-prop-2-ylester (II-4),
(5-Chlor-8-chinolinoxy)essigsäureethylester (II-5),
(5-Chlor-8-chinolinoxy)essigsäuremethylester (II-6),
(5-Chlor-8-chinolinoxy)essigsäureallylester (II-7),
(5-Chlor-8-chinolinoxy)essigsäure-2-(2-propyliden-iminoxy)-1-ethylester (II-8),
(5-Chlor-8-chinolinoxy)essigsäure-2-oxo-prop-1-ylester (II-9),
(5-Chlor-8-chinolinoxy)essigsäure (II-10) und dessen Salze wie sie z.B. in der WO-A-2002/34048 beschrieben sind, und verwandte Verbindungen, wie sie in EP-A-0 860 750, EP-A-0 094 349 und EP-A-0 191 736 oder EP-A-0 492 366 beschrieben sind.

f) Verbindungen vom Typ der (5-Chlor-8-chinolinoxy)-malonsäure, d.h. der Formel (II), worin $(R^{19})_{n'}$ = 5-Cl, $R^{20}$= $OR^{24}$, T = -CH(COO-Alkyl)- ist, vorzugsweise die Verbindungen (5-Chlor-8-chinolinoxy)-malonsäurediethylester (II-11), (5-Chlor-8-chinolinoxy)-malonsäurediallylester, (5-Chlor-8-chinolinoxy)-malonsäure-methyl-ethylester und verwandte Verbindungen, wie sie in EP-A-0 582 198 beschrieben sind.

g) Verbindungen vom Typ der Dichloracetamide, d.h. der Formel (III), vorzugsweise:

N,N-Diallyl-2,2-dichloracetamid (Dichlormid (III-1), aus US-A 4,137,070), 4-Dichloracetyl-3,4-dihydro-3-methyl-2H-1,4-benzoxazin (IV-2, Benoxacor, aus EP 0 149 974),
N1,N2-Diallyl-N2-dichloracetylglycinamid (DKA-24 (III-3), aus HU 2143821), 4-Dichloracetyl-1-oxa-4-aza-spiro [4,5]decan (AD-67),
2,2-Dichlor-N-(1,3-dioxolan-2-ylmethyl)-N-(2-propenyl)acetamid (PPG-1292),
3-Dichloracetyl-2,2,5-trimethyloxazolidin (R-29148, III-4), 3-Dichloracetyl-2,2-dimethyl-5-phenyloxazolidin,
3-Dichloracetyl-2,2-dimethyl-5-(2-thienyl)oxazolidin,
3-Dichloracetyl-5-(2-furanyl)-2,2-dimethyloxazolidin (Furilazole (III-5), MON 13900),
1-Dichloracetyl-hexahydro-3,3,8a-trimethylpyrrolo[1,2-a]pyrimidin-6(2H)-on (Dicyclonon, BAS 145138),

h) Verbindungen der Gruppe (b), vorzugsweise
1,8-Naphthalsäureanhydrid (b-1),
Methyl-diphenylmethoxyacetat (b-2),
Cyanomethoxyimino(phenyl)acetonitril (Cyometrinil) (b-3),
1-(2-Chlorbenzyl)-3-(1-methyl-1-phenylethyl)harnstoff (Cumyluron) (b-4),
O,O-Diethyl S-2-ethylthioethyl phosphordithioat (Disulfoton) (b-5),
4-Chlorphenyl-methylcarbamat (Mephenate) (b-6),
O,O-Diethyl-O-phenylphosphorotioat (Dietholate) (b-7),
4-Carboxy-3,4-dihydro-2H-1-benzopyran-4-essigsäure (CL-304415, CAS-Regno: 31541-57-8) (b-8),
1,3-Dioxolan-2-ylmethoxyimino(phenyl)acetonitril (Oxabetrinil) (b-9),
4'-Chlor-2,2,2-trifluoracetophenon O-1,3-dioxolan-2-ylmethyloxim (Fluxofenim) (b-10),
4,6-Dichlor-2-phenylpyrimidin (Fenclorim) (b-11),
Benzyl-2-chlor-4-trifluormethyl-1,3-thiazol-5-carboxylat (Flurazole) (b-12),
2-Dichlormethyl-2-methyl-1,3-dioxolan (MG-191) (b-13),
N-(4-Methylphenyl)-N'-(1-methyl-1-phenylethyl)harnstoff (Dymron) (b-14), (2,4-Dichlorphenoxy)essigsäure (2,4-D),
(4-Chlorphenoxy)essigsäure,
(R,S)-2-(4-Chlor-o-tolyloxy)propionsäure (Mecoprop),
4-(2,4-Dichlorphenoxy)buttersäure (2,4-DB),
(4-Chlor-o-tolyloxy)essigsäure (MCPA),
4-(4-Chlor-o-tolyloxy)buttersäure,
4-(4-Chlorphenoxy)buttersäure,
3,6-Dichlor-2-methoxybenzoesäure (Dicamba),
1-(Ethoxycarbonyl)ethyl 3,6-dichlor-2-methoxybenzoat (Lactidichlor) sowie deren Salze und Ester, vorzugsweise ($C_1$-$C_8$).

Bevorzugt sind weiterhin als Safener bekannte Verbindungen der Formel (IV) oder deren Salze, worin

$R^{30}$ Wasserstoff, ($C_1$-$C_6$)-Alkyl, ($C_3$-$C_6$)-Cycloalkyl, Furanyl oder Thienyl, wobei jeder der letztgenannten 4 Reste unsubstituiert oder durch einen oder mehrere Substituenten aus der Gruppe Halogen, ($C_1$-$C_4$)-Alkoxy, Halogen-($C_1$-$C_6$)-alkoxy und ($C_1$-$C_4$)-Alkylthio und im Falle cyclischer Reste auch ($C_1$-$C_4$)-Alkyl und ($C_1$-$C_4$)-Haloalkyl substituiert ist,
$R^{31}$ Wasserstoff,
$R^{32}$ Halogen, Halogen-($C_1$-$C_4$)-alkyl, Halogen-($C_1$-$C_4$)-alkoxy, ($C_1$-$C_4$)-Alkyl, ($C_1$-$C_4$)-Alkoxy, ($C_1$-$C_4$)-Alkylsulfonyl, ($C_1$-$C_4$)-Alkoxycarbonyl oder ($C_1$-$C_4$)- Alkylcarbonyl, vorzugsweise Halogen, ($C_1$-$C_4$)-Halogenalkyl, wie Trifluormethyl, ($C_1$-$C_4$)- Alkoxy, Halogen-($C_1$-$C_4$)-alkoxy, ($C_1$-$C_4$)-Alkoxycarbonyl oder ($C_1$-$C_4$)- Alkylsulfonyl,
$R^{33}$ Wasserstoff,
$R^{34}$ Halogen, ($C_1$-$C_4$)-Alkyl, Halogen-($C_1$-$C_4$)-alkyl, Halogen-($C_1$-$C_4$)-alkoxy, ($C_3$-$C_6$)-Cycloalkyl, Phenyl, ($C_1$-$C_4$)-Alkoxy, Cyano, ($C_1$-$C_4$)-Alkylthio, ($C_1$-$C_4$)- Alkylsulfinyl, ($C_1$-$C_4$)-Alkylsulfonyl, ($C_1$-$C_4$)-Alkoxycarbonyl oder ($C_1$-$C_4$)-Alkylcarbonyl, vorzugsweise Halogen, ($C_1$-$C_4$)-Alkyl, ($C_1$-$C_4$)-Halogenalkyl, wie Trifluormethyl, Halogen-($C_1$-$C_4$)-alkoxy, ($C_1$-$C_4$)-Alkoxy oder ($C_1$-$C_4$)-Alkylthio,
n 0, 1 oder 2 und
m 1 oder 2 bedeuten.

Besonders bevorzugt sind als Safener bekannte Verbindungen der Formel (IV), worin
$R^{30}$ = $H_3C$-O-$CH_2$-, $R^{31}$ = $R^{33}$ = H, $R^{34}$ = 2-OMe ist (IV-1),
$R^{30}$ = $H_3C$-O-$CH_2$-, $R^{31}$ = $R^{33}$ = H, $R^{34}$ = 2-OMe-5-Cl ist (IV-2),
$R^{30}$ = Cyclopropyl, $R^{31}$ = $R^{33}$ = H, $R^{34}$ = 2-OMe ist (IV-3),
$R^{30}$ = Cyclopropyl, $R^{31}$ = $R^{33}$ = H, $R^{34}$ = 2-OMe-5-Cl ist (IV-4),
$R^{30}$ = Cyclopropyl, $R^{31}$ = $R^{33}$ = H, $R^{34}$ = 2-Me ist (IV-5),
$R^{30}$ = tert. Butyl, $R^{31}$ = $R^{33}$ = H, $R^{34}$ = 2-OMe ist (IV-6).

Weiterhin bevorzugt sind als Safener bekannte Verbindungen der Formel (V), in der

$X^3$ CH;

$R^{35}$ Wasserstoff, ($C_1$-$C_6$)-Alkyl, ($C_3$-$C_6$)-Cycloalkyl, ($C_2$-$C_6$)-Alkenyl, ($C_5$-$C_6$)- Cycloalkenyl, Phenyl oder 3- bis 6-gliedriges Heterocyclyl mit bis zu drei Heteroatomen aus der Gruppe Stickstoff, Sauerstoff und Schwefel, wobei die sechs letztgenannten Reste gegebenenfalls durch einen oder mehrere, gleiche oder verschiedene Substituenten aus der Gruppe Halogen, ($C_1$-$C_6$)- Alkoxy, ($C_1$-$C_6$)-Haloalkoxy, ($C_1$-$C_2$)-Alkylsulfinyl, ($C_1$-$C_2$)-Alkylsulfonyl, ($C_3$-$C_6$)-Cycloalkyl, ($C_1$-$C_4$)-Alkoxycarbonyl, ($C_1$-$C_4$)-Alkylcarbonyl und Phenyl und im Falle cyclischer Reste auch ($C_1$-$C_4$)-Alkyl und ($C_1$-$C_4$)-Haloalkyl substituiert sind;

$R^{36}$ Wasserstoff, ($C_1$-$C_6$)-Alkyl, ($C_2$-$C_6$)-Alkenyl, ($C_2$-$C_6$)-Alkinyl, wobei die drei letztgenannten Reste gegebenenfalls durch einen oder mehrere, gleiche oder verschiedene Substituenten aus der Gruppe Halogen, Hydroxy, ($C_1$-$C_4$)-Alkyl, ($C_1$-$C_4$)-Alkoxy und ($C_1$-$C_4$)-Alkylthio substituiert sind;

$R^{37}$ Halogen, ($C_1$-$C_4$)-Haloalkyl, ($C_1$-$C_4$)-Haloalkoxy, Nitro, ($C_1$-$C_4$)-Alkyl, ($C_1$-$C_4$)- Alkoxy, ($C_1$-$C_4$)-Alkylsulfonyl, ($C_1$-$C_4$)-Alkoxycarbonyl oder ($C_1$-$C_4$)- Alkylcarbonyl;

$R^{38}$ Wasserstoff;

$R^{39}$ Halogen, Nitro, ($C_1$-$C_4$)-Alkyl, ($C_1$-$C_4$)-Haloalkyl, ($C_1$-$C_4$)-Haloalkoxy, ($C_3$-$C_6$)- Cycloalkyl, Phenyl, ($C_1$-$C_4$)-Alkoxy, Cyano, ($C_1$-$C_4$)-Alkylthio, ($C_1$-$C_4$)- Alkylsulfinyl, ($C_1$-$C_4$)-Alkylsulfonyl, ($C_1$-$C_4$)-Alkoxycarbonyl oder ($C_1$-$C_4$)-Alkylcarbonyl;

n 0, 1 oder 2 und

m 1 oder 2

bedeuten.

Bevorzugte als Safener bekannte Verbindungen der Formel (VI) sind (S3-1), (S3-2), (S3-3), (S3-4) und (S3-5).

Bevorzugt sind auch Verbindungen der Formel (VII) sind
1-[4-(N-2-Methoxybenzoylsulfamoyl)phenyl]-3-methylharnstoff (VII-1),
1-[4-(N-2-Methoxybenzoylsulfamoyl)phenyl]-3,3-dimethylharnstoff (VII-2),
1-[4-(N-4,5-Dimethylbenzoylsulfamoyl)phenyl]-3-methylharnstoff (VII-3) und
1-[4-(N-Naphthoylsulfamoyl)phenyl]-3,3-dimethylharnstoff (VII-4).

Ebenfalls bevorzugt sind Verbindungen der Formeln VIII-1 bis VIII-4

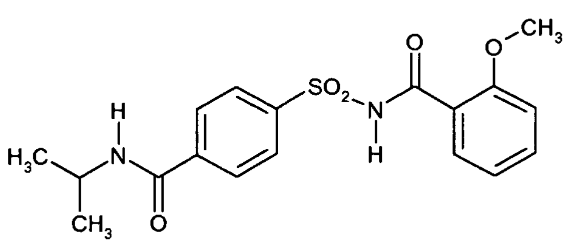

VIII-1

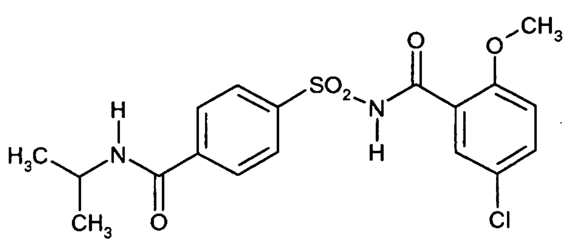

VIII-2

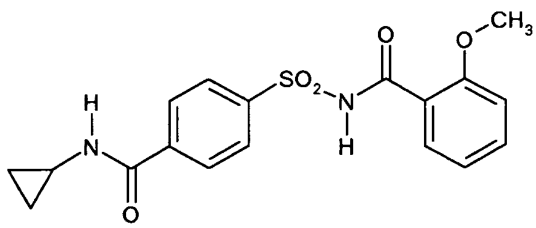

VIII-3

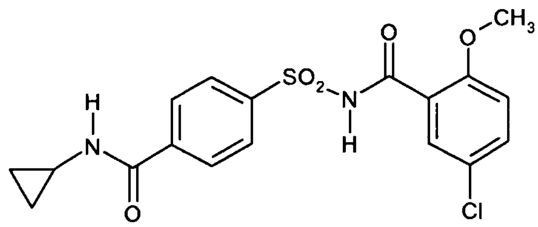

VIII-4

von denen wiederum die Verbindung VIII-3 (4-Cyclopropylaminocarbonyl-N-(2-methoxybenzoyl)benzolsulfonamide) zur Verwendung als Mittel zur Toleranzerhöhung bei Pflanzen gegenüber abiotischen Stressoren ganz besonders bevorzugt ist.

Besonders bevorzugte Verbindungen zur Verwendung als Mittel zur Toleranzerhöhung bei Pflanzen gegenüber abiotischen Stressoren sind solche, die ausgewählt sind aus der Gruppe von als Safener bekannten Verbindungen bestehend aus den Verbindungen der Formeln I-1 (Mefenpyr-diethyl), I-9 (Isoxadifen-ethyl), II-1 (Chloquintocet-mexyl), b-11 (Fenclorim), b-14 (Dymron), und VIII-3 (4-Cyclopropylaminocarbonyl-N-(2-methoxybenzoyl)benzolsulfonamide, ganz besonders bevorzugt sind die Verbindungen I-1 und VIII-3).

Die zuvor identifzierten/genannten unter Umständen bereits als Safener bekannten Verbindungen können auch in gentechnisch veränderten Pflanzen eingesetzt werden.

Die gentechnisch veränderten (auch transgen genannt) Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der identifizierten/genannten als Safener bekannten Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten, besonders bevorzugt in Kulturen von Mais, Weizen, Gerste, Roggen, Hafer, Reis, Raps, Zuckerrübe und Soja, ganz besonders bevorzugt in Kulturen von Mais, Weizen, Reis, Raps, Zuckerrübe und Soja.

Daneben können auch transgene Pflanzen mit mit Hilfe von DNA-Microarrays identifizierten Substanzen wie auch der bereits als Safener bekannten Moleküle behandelt werden, deren Toleranz gegenüber abiotischen Stressoren bereits durch gentechnische Maßnahmen erhöht worden ist, so dass eine synergistische Wirkung der endogen kodierten Toleranz und der exogen applizierten toleranzsteigernden Wirkung beobachtet wird.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen

- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die mit Hilfe der DNA-Microarrays identifizierten oder aber als Safener bekannten Moleküle in transgenen Kulturen eingesetzt werden, welche gegen Herbizide aus der Gruppe der Sulfonylharnstoffe, Glufosinate-ammonium oder Glyphosate-isopropylammonium und analoge Wirkstoffe resistent sind und/oder auf der Basis gentechnischer Veränderung eine endogene Toleranz gegenüber abiotischen Stressoren aufweisen.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Die Verwendung der mit DNA-Microarrays identifizierten Verbindungen bzw. von bereits als Safener bekannten Verbindungen zur Erhöhung der Toleranz gegenüber abiotischen Stressoren in transgenen Kulturpflanzen, bevorzugt mit dem Ziel der Ertragssteigerung ist gezeigt.

Ein Verfahren zum Auffinden einer die Toleranz gegenüber abiotischen Stressoren bei Pflanzen erhöhenden Verbindung, wobei die Steigerung der Transkription bzw. Expression einzelner oder mehrerer, pflanzenendogener Gene wie beispielsweise Genen codierend für Proteine aus der Gruppe der Cytrochrom-Oxidasen, wie der Cytochromoxidase P450, Glycosyltransferasen, Uricasen, wie der Uricase II (E.C.17.3.3), Peptidasen, verschiedener Membranproteine, Amidohydrolasen, sowie verschiedener allgemeiner Stressproteine, als Indiz für die Induktion bewertet wird, ist gezeigt.

Ein Verfahren zum Auffinden von Verbindungen, die die Transkription der für pflanzenendogene Enzyme der Stresstoleranz kodierenden Gene induzieren, dadurch gekennzeichnet, dass:

a) Testpflanzen einem oder mehreren abiotischen Stressoren ausgesetzt werden,

b) Kontrollpflanzen, unter ansonsten gleichen Bedingungen wie Testpflanzen unter a), zusätzlich mit einer zu testenden Verbindung in Kontakt gebracht werden, sei es in Form gebeizten Saatgutmaterials, oder der Besprühung zu einem bestimmten Entwicklungszeitpunkt oder durch Wurzelaufnahme,

c) RNA aus den Test- und Kontrollpflanzen extrahiert wird,

d) die RNA entweder direkt radioaktiv oder nicht radioaktiv markiert wird, oder aber die RNA unter gleichzeitiger enzymatischer Umschreibung in die korrespondierende cDNA radioaktiv oder nicht-radioaktiv markiert wird, oder aber die erhaltene, nicht markierte cDNA enzymatisch in eine korrespondierende radioaktive oder nicht-radioaktive markierte cRNA umgeschrieben wird,

e) ein pflanzliche DNA-Sequenzen enthaltender DNA-Microarray mit den gemäß Schritt d) erhaltenen Substanzen hybridisiert wird,

f) Expressionsprofile der Gene für die Expression verschiedener Stressproteine vergleichend für die gemäß a) und b) getesteten Pflanzen erstellt werden,

g) eine Quantifizierung der gemäß f) gemessenen Expressionsunterschiede erfolgt, und

h) eine abschließende Systematisierung der gemäß g) zugeordneten Expressionsprofile durch Cluster-Analyse erfolgt,

ist hier beinhaltet.

Im Fall des zuvor genannten Schritts d) ist die enzymatische Umschreibung der erhaltenen cDNA in eine cRNA als bevorzugter Verfahrensschritt anzusehen, da hierdurch eine nochmalige Amplifikation der Hybridisierungsprobe erreicht werden kann. Ebenso bevorzugt ist die Markierung mittels nicht radioaktiver Nukleotide, besonders bevorzugt die Markierung mittels biotinyliertem UTP und/oder CTP, wobei der Nachweis im Anschluss an die erfolgte Hybridisierungsreaktion durch Bindung von Streptavidin-Phycoerythrin als Fluorophor and die biotinylierte cRNA erfolgt. Ein Nachweis der spezifischen Phycoerythrin-Fluoreszenz, die als Grundlage für die Quantifizierung der gemessenen Expressionsunterschiede dient, erfolgt im Anschluss an die Hybridisierung mit Hilfe eines Laser-Scanners.

Bevorzugter Gegenstand ist ein Verfahren unter Einhalten der zuvor genannten Verfahrensabläufe a) - h), wobei im Fall der beabsichtigten Steigerung bei Hitzestress die Gene für die Expression der Cytrochrom-Oxidasen, wie der Cytochromoxidase P450, Glycosyltransferasen, Uricasen, wie der Uricase II (E.C.17.3.3), Peptidasen, verschiedener Membranproteine, Amidohydrolasen bei hitzegestressten und nicht hitzegestressten Pflanzen verglichen wird, bevorzugt der Gene die für die Expression der "N-carbamyl-L-amino acid Amidohydrolase" (Zm.11840.1.A1_at), der "Serine Carboxypeptidase (Zm.18994.2.A1_a_at), der Uricase II (E.C.1.7.3.3) und der Glycosyltransferase (Zm.12587.1.S1_s_at), ganz besonders bevorzugt der Gene für die Expression der "N-carbamyl-L-amino acid Amidohydrolase" (Zm. 11840.1.A1_at) und der "Serine Carboxypeptidase" (Zm.18994.2.A1_a_at) (Signatur gemäß Mais Genom-Array der Firma Affymetrix (Affymetrix Inc., 3380 Central Expressway, Santa Clara, CA, USA))
und wobei die Genexpression gegenüber einer hitzegestressten Kontrollpflanze bei Behandlung mit beispielsweise um den Faktor 1.5 oder mehr, vorzugsweise um den Faktor 1.5 bis 30, bevorzugt 1.5 bis 20, besonders bevorzugt 1.5 bis 10, ganz besonders bevorzugt 1.5 bis 5, erhöht ist, wobei die Steigerung der geänderten Expressionsprofile der einzelnen Gene unabhängig voneinander in den unterschiedlichen zuvor benannten Größenbereichen liegen kann.

Ebenfalls bevorzugter Gegenstand ist ein Verfahren unter Einhalten der zuvor genannten Verfahrensabläufe a) - h), wobei im Fall der beabsichtigten Steigerung bei Trockenstress beispielsweise die Gene für die Expression des Late Embryogenesis Abundant Proteinen, wie den Dehydrinen, des Universal Stress Proteins (Zm.818.1.A1_at), Non-symbiotic hemoglobin (Zm.485.1.A1_at), des Proteins mit der Adressierung "Zm.818.2.A1_a_at" (Mais Genom-Array der Firma Affymetrix (Affymetrix Inc., 3380 Central Expressway, Santa Clara, CA, USA)) und des Proteins mit der Adressierung "Zm.18682.1.A1_s_at" (Mais Genom-Array der Firma Affymetrix (Affymetrix Inc., 3380 Central Expressway, Santa Clara, CA, USA)) bei durch Trockenheit gestressten und nicht Trockenheit gestressten Pflanzen verglichen wird, bevorzugt die Gene für die Expression des Universal Stress Proteins (Zm.818.1.A1_at), Non-symbiotic hemoglobin (Zm.485.1.A1_at), des Proteins mit der Adressierung "Zm.818.2.A1_a_at" (Signatur gemäß Mais Genom-Array der Firma Affymetrix (Affymetrix Inc., 3380 Central Expressway, Santa Clara, CA, USA)) und des Proteins mit der Adressierung "Zm.18682.1.A1_s_at" (Mais Genom-Array der Firma Affymetrix (Affymetrix Inc., 3380 Central Expressway, Santa Clara, CA, USA)) und wobei die Genexpression gegenüber einer durch Trockenheit gestressten Kontrollpflanze bei Behandlung mit beispielsweise um den Faktor 1.5 oder mehr, vorzugsweise um den Faktor 1.5 bis 30, bevorzugt 1.5 bis 20, besonders bevorzugt 1.5 bis 10, ganz besonders bevorzugt 1.5 bis 8, erhöht ist, wobei die Steigerung der geänderten Expressionsprofile der einzelnen Gene unabhängig voneinander in den unterschiedlichen zuvor benannten Größenbereichen liegen kann.

Gegenstand ist weiter die Verwendung bestimmter DNA-Microarrays, die auf der Basis genetischer Informationen aus Pflanzen, bevorzugt genetischer Information aus Nutzpflanzen, besonders bevorzugt aus Nutzpflanzen wie beispielsweise aus Mais, Getreide, wie Weizen, Gerste, Roggen, Hafer, Reis und Soja, bevorzugt aus Mais, Weizen, Gerste, Roggen, Reis und Soja, besonders bevorzugt aus Gerste, Mais, Weizen, Reis und Soja, ganz besonders bevorzugt aus Mais, Weizen und Soja zur Auffindung von geänderten Genexpressionsmustern benutzt werden. Dabei werden die relativen Veränderungen der Genmuster für Gene verschiedener Stressproteine in mit den zu testenden Verbindungen behandelten Pflanzen im Vergleich zu unbehandelten Kontrollpflanzen unter ansonsten identischen Stressbedingungen betrachtet.

Gegenstand ist außerdem die Verwendung der Promotoren der beschriebenen Indikatorgene in Verbindung mit speziellen Reportergenen (z.B. GUS, GFP, Luciferase etc) zum Auffinden von Substanzen mit positiver Wirkung auf die abiotische Stresstoleranz in Kulturpflanzen. Dabei werden transgene Testpflanzen erzeugt, welche die erwähnten Promoter-Reportergen-Konstrukte enthalten. Wirkstoffe welche die abiotische Stresstoleranz von Pflanzen nach dem beschriebenen Mechanismus erhöhen, induzieren die Expression des Reportergens und können mit Hilfe eines kolorimetrischen, fluorimetrischen oder sonstigen hierfür geeigneten Assays identifiziet werden.

Gegenstand ist weiterhin die Verwendung der beschriebenen Indikatorgene zur Steigerung der abiotischen

Stresstoleranz in transgenen Kulturpflanzen. Dabei werden die Gene mit einem geeigneten Promoter, welcher die gewünschte Stärke und Spezifität besitzt fusioniert und die Konstrukte in monocotyle oder dicotyle Kulturpflanzen transformiert. Die erzeugten transgenen Pflanzen zeichnen sich durch eine erhöhte Toleranz gegen abiotischen Stress, z.B. Kälte, Hitze, Trockenheit etc. aus.

Weiterer Gegenstand ist auch die Verwendung der Verbindungen die mit Hilfe des DNA-Microarrays unter Betrachtung der Expressionsprofile der Gene der identifiziert wurden und/oder bereits als Safener bekannter Verbindungen, die im Falle von abiotischen Stressbedingungen, wie beispielsweise gegenüber auf diese Pflanze einwirkenden abiotischem Stressoren, wie Temperatur (Kälte, Frost oder Hitze), Wasser (Trockenheit oder Dürre), oder die chemische Belastung (Mangel oder Überschuss an Mineralsalzen, Schwermetalle, gasförmige Noxen) positiv, d.h. expressionssteigernd hinsichtlich ihrer induktiven Wirkung auf einzelne oder mehrer Gene der pflanzendogenen Abwehrmechanismen wirken, wie beispielsweise im Fall von Hitzestress auf Cytrochrom-Oxidasen, wie der Cytochromoxidase P450, auf Glycosyltransferasen, auf Uricasen, wie der Uricase II (E.C.17.3.3), auf Peptidasen, auf verschiedener Membranprotein, auf Amidohydrolasen und/oder verschiedene Stressproteine und/oder beispielsweise im Fall von Trockenstress positiv, d.h. expressionssteigernd hinsichtlich ihrer induktiven Wirkung auf einzelne oder mehrere Gene der universeller Stressproteine, Non-symbiotic hemoglobin (Zm.485.1.A1_at), des Proteins mit der Adressierung "Zm.818.2.A1_a_at" (Mais Genom-Array der Firma Affymetrix (Affymetrix Inc., 3380 Central Expressway, Santa Clara, CA, USA)) und des Proteins mit der Adressierung "Zm.18682.1.A1_s_at" (Signatur gemäß Mais Genom-Array der Firma Affymetrix (Affymetrix Inc., 3380 Central Expressway, Santa Clara, CA, USA)) identifiziert wurden, als Wirkstoffe zur Steigerung der Stresstoleranz bei Nutzpflanzen.

Gegenstand ist auch die Verwendung von mit Hilfe des DNA-Microarrays identifizierten Substanzen wie auch der bereits als Safener bekannten Moleküle zur Toleranzerhöhung gegenüber abiotischen Stressoren in verschiedenen Kulturpflanzen, wie Mais, Getreide, wie Weizen, Gerste, Roggen, Hafer, Reis und Soja, bevorzugt Mais, Weizen, Gerste, Roggen, Reis und Soja, besonders bevorzugt Mais, Weizen, Reis und Soja, ganz besonders bevorzugt Mais, Weizen und Soja.

Gegenstand ist daher auch die Verwendung von Verbindungen, die mit Hilfe des DNA-Microarrays unter Betrachtung der Expressionsprofile der Gene identifiziert wurden und/oder bereits als Safener bekannter Verbindungen, die in Pflanzen, direkt oder indirekt, wie beispielsweise durch eine Signaltransduktionskette, zur Erhöhung der Toleranz gegenüber abiotischen Stressoren, wie beispielsweise Temperatur (wie Kälte, Frost oder Hitze), Wasser (wie Trockenheit, Dürre oder Anoxia), oder die chemische Belastung (wie Mangel oder Überschuss an Mineralsalzen. Schwermetallen, gasförmigen Noxen) beitragen, zur Ertragssteigerung, zur Verlängerung der Vegetationsperiode, zur Ermöglichung einer früheren Aussaat, zur Steigerung der Qualität, oder zur Verwendung im Rahmen der Züchtung unter Verwendung ansonsten weniger vitaler Inzucht-Linien.

Gegenstand ist daher auch ein Verfahren zur Ertragssteigerung in Nutzpflanzenkulturen, zur Verlängerung der Vegetationsperiode, zur Ermöglichung einer früheren Aussaat, zur Steigerung der Qualität, oder zur Verwendung im Rahmen der Züchtung unter Verwendung ansonsten weniger vitaler Inzucht-Linien dadurch gekennzeichnet, dass die Nutzpflanzen durch Saatgut-Beizung, durch Blattspritzung oder durch Bodenapplikation mit einem oder mehreren Verbindungen, die mit Hilfe des DNA-Microarrays identifiziert wurden, und/oder bereits als Safener bekannten Verbindung behandelt werden.

Bevorzugt sind hierbei solche Verbindungen, die in ihrer Verwendung als sogenannte Safener bereits im Pflanzenschutz bekannt sind, wie beispielsweise aus der Gruppe der als Safener bekannten Verbindungen bestehend aus den Verbindungen der Formeln I-1 (Mefenpyr-diethyl), I-9 (Isoxadifen-ethyl), II-1 (Chloquintocet-mexyl), b-11 (Fenclorim), b-14 (Dymron), VIII-3 (4-Cyclopropylaminocarbonyl-N-(2-methoxybenzoyl)benzolsulfonamide), ganz besonders bevorzugt sind die Verbindungen I-1 und VIII-3 (4-Cyclopropylaminocarbonyl-N-(2-methoxybenzoyl) benzolsulfonamide)).

Durch einzelne oder kombinierte Applikation der zuvor genannten Verbindungen können Nutzpflanzen effektiv gegenüber den Auswirkungen abiotischer Stressoren geschützt werden, was sich z.B. auch in höheren Erträgen niederschlägt.

Gegenstand ist daher auch ein Verfahren Erhöhung der Toleranz von Nutzpflanzen in Nutzpflanzenkulturen gegenüber abiotischen Stressoren durch einzelne oder kombinierte Applikation der mit Hilfe des DNA-Microarrays unter Betrachtung der Expressionsprofile der Gene der identifizierten Verbindungen und/oder bereits als Safener bekannter Verbindungen.

Die folgenden Beispiele beschreiben die Erfindung im einzelnen.

Beispiel 1

Nachweis der Wirkung von Safenern auf Pflanzen, die gezielten Trockenstressbedingungen ausgesetzt waren, durch Gene Expression Profiling (GEP):

## Abiotischer Stressor = Trockenstress

Mais-Samen der Sorte Lorenzo wurden mit der Verbindung 4-Cyclopropylaminocarbonyl-N-(2-methoxybenzoyl)benzolsulfonamide (= VIII-3) gebeizt.

Dazu wurden 10 g Samen mit 20 mg Wirkstoff gelöst in 2 ml Methylenchlorid unter leichtem Schütteln inkubiert, bis das Lösungsmittel abgedampft war (ca. 30 min.). Die Samen der Kontrollgruppe wurden lediglich mit Lösungsmittel gebeizt. Anschließend wurden die behandelten Samen in Töpfe mit Erde (Durchmesser: 10 cm, je 10 Samen pro Topf) ausgelegt und die Maiskeimlinge für 10 Tage in einer Klimakammer unter definierten Licht-, Feuchtigkeits- und Temperaturbedingungen [Weißlicht, Langtag (16 h hell, 8 h dunkel), 70 % Luftfeuchtigkeit, 24˚C] angezogen. Es wurden je 2 x 10 Töpfe für die Kontrollgruppen und für den Trockenstress-Versuch verwendet. Während der Anzucht wurden die Pflanzen alle 2 Tage durch Anstauen von unten in einer Wanne für 20 min. bewässert. 10 Tage nach Auskeimen der Samen wurden die Maispflanzen dem Trockenstress ausgesetzt. Dazu wurden die Pflanzen der Kontrollgruppe 1 (ohne Wirkstoff-Beizung) und der Testgruppe (mit Wirkstoff-Beizung) nur noch alle 7 Tage wie oben beschrieben gewässert. Bei den Pflanzen der Kontrollgruppe 2 (ohne Wirkstoff-Beizung) und der Testgruppe 2 (mit Wirkstoff-Beizung) wurde das normale Bewässerungsschema beibehalten. Nach 3 Wochen Trockenstress-Bedingungen wurde der Versuch wie folgt ausgewertet. Die oberirdischen Pflanzenteile wurden abgeschnitten und über Nacht bei 50˚C getrocknet. Am nächsten Tag wurde die Blattmasse pro Topf (Trockenmasse) in [g] bestimmt.

Die Messwerte wurden über die jeweils 10 Töpfe der Pflanzengruppe gemittelt. Die in der Tabelle 1 angegebenen Zahlenwerte sind Relativwerte in [%] bezogen auf die Messergebnisse der Kontrollgruppe 2 (ohne Wirkstoff-Beizung, normales Bewässerungsschema).

Tabelle 1: Trockenstress-Versuch mit Maispflanzen ohne und mit Wirkstoff-Beizung

| Pflanzengruppe: | Behandlung: | Rel. Trockenmasse [%]: |
|---|---|---|
| Kontrollgruppe 1 | -S/ +T | 50 |
| Kontrollgruppe 2 | -S/ -T | 100 |
| Testgruppe 1 | +S/ +T | 80 |
| Testgruppe 2 | +S/ -T | 100 |
| S = Verbindung VIII-3 (= 4-Cyclopropylaminocarbonyl-N-(2-methoxybenzoyl) benzolsulfonamide),<br>T = Trockenstress | | |

Die durchschnittliche Trockenmasse war bei Pflanzen aus ungebeizten und aus gebeizten Samen ohne Stressbedingungen gleich (Kontrollgruppe 2, Testgruppe 2).

Die Pflanzen aus der Gruppe mit Beizung mit der Verbindung VIII-3 (= 4-Cyclopropylaminocarbonyl-N-(2-methoxybenzoyl)benzolsulfonamide) zeigten im Durchschnitt einen kompakteren Habitus als die Pflanzen der Kontrollgruppe, der sich aber nicht auf die Trockenmasse auswirkte. Unter Trockenstress war die durchschnittliche Blattmasse (Trockenmasse) der Wirkstoff-gebeizten Pflanzen gegenüber den ungebeizten Kontrollpflanzen jedoch signifikant erhöht (Kontrollgruppe 1, Testgruppe 1).

Beispiel 2

Abiotischer Stressor = Hitzestress

Mais-Samen der Sorte Lorenzo wurden wie in Beispiel 1 mit der Verbindung 4-Cyclopropylaminocarbonyl-N-(2-methoxybenzoyl)benzolsulfonamide (=VIII-3) gebeizt bzw. nur mit Lösungsmittel ohne Wirkstoff behandelt. Die Anzucht der Keimlinge erfolgte für 10 Tage in der Klimakammer unter definierten Bedingungen ebenfalls wie in Beispiel 1 beschrieben. Für den Hitzestress-Versuch wurden 2 x 10 Töpfe mit Maispflanzen verwendet. Die Kontrollgruppe bestand aus ungebeizten Pflanzen (Lösungsmittel), die Testgruppe aus Wirkstoff-gebeizten Pflanzen. Zur Applizierung der Hitzestress-Bedingungen wurden beide Pflanzengruppen für 2 Tage in einen Klimaschrank bei 45˚C, Weißlicht, Langtag (16 h hell, 8 h dunkel) und 70 % Luftfeuchtigkeit gestellt. Um eine Austrocknung durch die hohe Temperatur zu vermeiden, wurden die Pflanzen 1 x pro Tag durch Anstauen von unten in einer Wanne bewässert. Nach dem Hitzestress konnte beobachtet werden, dass - besonders in der Kontrollgruppe - die Sprosse vieler Pflanzen umgeknickt

waren und die Blätter flach auf dem Boden lagen.

Der Versuch wurde nach folgenden Kriterien quantitativ ausgewertet.

Nach der Hitze-Behandlung wurden die umgeknickten Pflanzen ausgezählt und das Ergebnis pro Topf bewertet:

< 20 % der aufgelaufenen Pflanzen umgeknickt: schwache Schädigung O
20-50 % der aufgelaufenen Pflanzen umgeknickt: mittlere Schädigung
> 50 % der aufgelaufenen Pflanzen umgeknickt: starke Schädigung

Anschließend wurden alle Pflanzen für 2 Wochen unter Standard-Bedingungen weiter kultiviert. Dann wurde der Längenzuwachs der Einzelpflanzen gemessen und die Überlebensrate der Pflanzen pro Topf bestimmt:

> 50 % Überlebensrate: schwache Schädigung ○

20-50 % Überlebensrate: mittlere Schädigung ◍

< 20 % Überlebensrate: starke Schädigung ●

Die Ergebnisse der Versuchsauwertung sind in Tabelle 2 zusammengefasst.

Die ungebeizten Kontrollpflanzen wurden durch den Hitzestress stark geschädigt. Auffallend waren insbesondere das Umknicken der Sprosse bei den meisten Pflanzen, sowie die geringe Überlebensrate. Die Wirkstoff-gebeizten Testpflanzen zeichneten sich besonders durch ein wesentlich besseres "Standing" aus. In der Endbonitur wurde zwar auch bei diesen Pflanzen die Schädigung durch den starken Hitzestress deutlich, die Überlebensrate war aber signifikant höher als in der Kontrollgruppe.

Tabelle 2: Trockenstress-Versuch mit Maispflanzen ohne und mit Wirkstoff-Beizung

| Pflanzengruppe: | Behandlung: | |
|---|---|---|
| Kontrollgruppe | -S/ +H | Zwischenbonitur (umgeknickte Pflanzen): |
| | | ●●●●●●●○○○ |
| | | Endbonitur (Überlebensrate): |
| | | ●●●●●●●●● ○ |
| Testgruppe | +S/ +H | Zwischenbonitur (umgeknickte Pflanzen): |
| | | ○○○○○○○○○○ |
| | | Endbonitur (Überlebensrate): |
| | | ●●●●● |
| S = Verbindung VIII-3 (= 4-Cyclopropylaminocarbonyl-N-(2-methoxybenzoyl)benzolsulfonamide), H = Hitzestress | | |

Beispiel 3

Abiotischer Stressor = Kältestress (Gewächshaus)

Mais-Samen der Sorte Lorenzo wurden in 10 cm Töpfen in Erde mit je 10 Samen pro Topf ausgesät. Alle Versuchsgruppen bestanden aus jeweils 4 Töpfen. Die ausgesäten Samen der Testgruppen 1 und 2 wurden im Vorauflauf mit 50 bzw. 100 [g a.i./ha] der Verbindung 4-Cyclopropylaminocarbonyl-N-(2-methoxybenzoyl) Benzolsulfonamide (= VIII-3) gespritzt. Die Samen der Kontrollgruppe blieben unbehandelt. Die Pflanzen wurden unter kontrollierten Bedingungen in der Klimakammer [Weißlicht (Langtag: 16 h hell, 8 h dunkel), 22˚C Tagestemperatur, 14˚C Nachttemperatur, 60 % Luftfeuchtigkeit] angezogen.

Nach der Keimung, wenn die Pflanzen eine Höhe von ca. 1 cm erreicht hatten, wurden 2 Töpfe aus jeder Gruppe für 6 h in einer anderen Klimakammer unter Kältestress-Bedingungen bei -2˚ C inkubiert. Anschließend wurden diese Pflanzen wieder zu den anderen in die erste Klimakammer gestellt.

Nach weiteren 24 h unter Standard-Bedingungen wurde der Versuch ausgewertet. Es konnte beobachtet werden, dass der Kältestress Chlorosen an den Blattspitzen der Keimlinge der unbehandelten Kontrollgruppe hervorrief.

Diese Symptome waren an den Wirkstoff-behandelten Pflanzen gar nicht oder nur sehr eingeschränkt zu beobachten.

Alle Pflanzen der Testgruppen und der Kontrollgruppe, die ausschließlich unter Standard-Bedingungen ohne Kältestress gehalten wurden, zeigten keinerlei Schadsymptome.

Zur quantitativen Auswertung des Versuchs wurden die Pflanzen mit Aufhellungen an den Blattspitzen ausgezählt. Die Gesamtzahl der Pflanzen pro Versuchsgruppe und Kältestress-Behandlung war 20 verteilt auf je 2 Töpfe.

Die Ergebnisse der Versuchsauswertung sind in Tabelle 3 zusammengefasst.

Tabelle 3: Kältestress-Versuch (Gewächshaus) mit Maispflanzen ohne und mit Wirkstoff-Behandlung mit der Verbindung VIII-3 (= 4-Cyclopropylaminocarbonyl-N-(2-methoxybenzoyl)benzolsulfonamide) im Vorauflauf. Alle Pflanzen waren Kältestress-Behandlung ausgesetzt. Die Gesamtzahl de Pflanzen pro Gruppe betrug 20.

| Pflanzengruppe: | Wirkstoff-Behandlung (Vorauflauf) [g a.i./ha]: | Anzahl der geschädigten Pflanzen (Chlorosen): |
|---|---|---|
| Kontrollgruppe | 0 | 9 |
| Testgruppe 1 | 50 | 1 |
| Testgruppe 2 | 100 | 0 |

Die Ergebnisse zeigen, dass die Behandlung mit Wirkstoff-Behandlung mit der Verbindung VIII-3 (= 4-Cyclopropylaminocarbonyl-N-(2-methoxybenzoyl)benzolsulfonamide) die durch Kältestress entstehenden Schadsymptome deutlich reduzieren kann, bzw. in der höheren Dosierung das Auftreten dieser Symptome gänzlich verhindert.

Beispiel 4

Abiotischer Stressor = Kältestress (Freiland)

Mais-Samen (Dent Corn) wurden mit 0,003 mg und 0,03 mg der Verbindung 4-Cyclopropylaminocarbonyl-N-(2-methoxybenzoyl)benzolsulfonamide (= VIII-3) pro g Samen gebeizt und auf je 34 m2 großen Testparzellen ausgesät. Eine Kontrollparzelle enthielt ungebeiztes Saatgut. Ca. 8 Tage nach Auflaufen der Saat waren die Keimlinge im Einblatt-Stadium und waren für 5 Tage den folgenden Temperatur-Bedingungen ausgesetzt:

| | Maximum: | Minimum: |
|---|---|---|
| 1. Tag: | 16,1˚C | 7,2˚C |
| 2. Tag: | 17,8˚C | 2,7˚C |
| 3. Tag: | 16,7˚C | 0,6˚C |
| 4. Tag: | 16,7˚C | 1,1˚C |
| 5. Tag: | 22,8˚C | 12,2˚C |

Nach dieser Kälteperiode wurden die Versuchsparzellen bonitiert. Dabei wurden alle Pflanzen einzeln bewertet und Pflanzen mit mindestens 20 % Kältesymptomen bezogen auf die gesamte Blattfläche (Verbrennungen und/ oder Chlorosen) als geschädigt bewertet.

Die Ergebnisse sind in der Tabelle 4 zusammengefasst. In der Kontrollparzelle ohne Wirkstoff-Beizung zeigten alle Pflanzen (100 %) die beschriebenen Kältesymptome. In den Testparzellen mit Wirkstoff-Beizung waren die Kälteschäden signifikant reduziert.

Hier konnten nur noch bei ca. 12 % der Pflanzen Schadsymptome beobachtet werden. Die maximale Frostschutzwirkung wurde im Größenbereich der in der Tabelle angegebenen Wirkstoff-Beizungsmengen erzielt.

Tabelle 4: Kältestress-Versuch (Freiland) mit Maispflanzen ohne und mit Wirkstoff-Beizung mit der Verbindung VIII-3 (= 4-Cyclopropylaminocarbonyl-N-(2-methoxybenzoyl) benzolsulfonamide)

| Pflanzengruppe: | Kälteschaden [%]*: |
|---|---|
| unbehandelt | 100 |
| Beizung mit 0,003 mg (VIII-3 = 4-Cyclopropylaminocarbonyl-N-(2-methoxybenzoyl) benzolsulfonamide) pro Samen | 12 |

(fortgesetzt)

| Pflanzengruppe: | Kälteschaden [%]*: |
|---|---|
| Beizung mit 0,03 mg VIII-3 (= 4-Cyclopropylaminocarbonyl-N-(2-methoxybenzoyl) benzolsulfonamide) pro Samen | 12 |
| *: Anteil der Pflanzen mit Kälteschaden > 20 % bezogen auf die Gesamtzahl der Pflanzen in der Testparzelle | |

Beispiel 5

Charakterisierung von Genen, die von Testsubstanzen unter abiotischen Stressbedingungen induziert werden, durch Gene Expression Profiling (GEP):

Mais-Samen der Sorte Lorenzo wurden wie in Beispiel 1 beschrieben mit der Verbindung VIII-3 (= 4-Cyclopropylaminocarbonyl-N-(2-methoxybenzoyl) benzolsulfonamide) bzw. mit Lösungsmittel gebeizt. Die Pflanzen wurden für 10 Tage in einer Klimakammer angezogen (Bedingungen: siehe Beispiel 1). Anschließend wurden die Pflanzen den folgenden Stress-Bedingungen ausgesetzt:

(1) Hitzestress: 6 h bei 45˚C
(2) Trockenstress: 7 Tage ohne Bewässerung , 24˚C

Die Kontrollpflanzen der jeweiligen Experimentgruppe wurden unter den in Beispiel 1 beschriebenen Standard-Bedingungen (Temperatur, Bewässerung) gehalten. Nach der Stressbehandlung wurden die Blätter der gestressten Pflanzen sowie der ungestressten Kontrollpflanzen geerntet, in Flüssig-Stickstoff schockgefroren und bis zur Aufarbeitung bei -80˚C gelagert. Alle Versuche wurden in Replikaten mit je 2 Töpfen durchgeführt.

Die Herstellung der markierten RNA-Sonden für die DNA-Chip-Hybridisierung erfolgte nach den Protokollen (Expression Analysis, Technical Manual) der Firma Affymetrix (Affymetrix Inc., 3380 Central Expressway, Santa Clara, CA, USA). Aus je 500 mg der geernteten Blätter wurde zunächst Gesamt-RNA isoliert. Je 10 μg Gesamt-RNA wurden für die Erst- und Zweitstrang cDNA-Synthese verwendet. Die cDNA wurde mit T7-Polymerase amplifiziert und dabei gleichzeitig mit Biotin-UTP markiert. Je 20 μg dieser biotinylierten cDNA wurden für die Hybridiserung des Mais Genom-Arrays von der Firma Affymetrix eingesetzt. Dieser DNA-Microarray enthält DNA-Sequenzen, die in ihrer Gesamtheit 13339 Gene repräsentieren. Anschließend wurden die DNA-Microarrays in der Affymetrix Fluidics Station gewaschen, mit Streptavidin/ Phycoerythrin (Molecular Probes, P/N S-866) gefärbt und mit dem zugehörigen Agilent Laser Scanner (Agilent Gene Array Scanner) gescannt. Die erhaltenen Fluoreszendaten wurden mit der Software Microarray Suite 5 von Affymetrix analysiert. Nach erfolgter Qualitätskontrolle wurden alle DNA-Chip-Analysen in einer Datenbank gespeichert. Zur Ermittlung von relativen Expressionswerten (Induktions-, Repressionsfaktoren) wurden die absoluten Expressionswerte der Gene aus den jeweiligen Stressexperimenten mit den Werten der jeweiligen Kontrollversuche (d.h., ohne abiotischen Stress und Beizung lediglich mit Lösungsmittel) verglichen und dabei die von der Affymetrix-Software vorgegebenen Signifikanz Kriterien zu Grunde gelegt. Die daraus erhaltenen je 4 Expressionswerte pro Gen wurden durch Medianberechnung gemittelt. Diese Mediane sind als Induktionsfaktoren in den Ergebnistabellen angegeben. Ähnlichkeitsvergleiche von Expressionsprofilen verschiedener Experimente und Cluster-Analysen wurden mit der Software "Genedata Expressionist" von Genedata (Genedata, Maulbeerstr. 46, CH-4016 Basel, Switzerland) durchgeführt.

Bei der Analyse der Expressionsprofile wurde speziell nach Genen gesucht, die durch die Testsubstanzen nur in Verbindung mit abiotischem Stress induziert werden, nicht aber durch die Substanzen oder durch Stress alleine. Solche Gene können als Indikatoren für zusätzliche Anti-Stress-Wirkungen der Substanzen angesehen werden, die über die bereits bekannte Safener-Wirkung hinausgehen. Die Ergebnisse der Analysen sind in den folgenden Tabellen aufgeführt.

Die Induktionsmuster der beschriebenen Indikatorgene erlauben das gezielte Auffinden von Wirkstoffen zur Steigerung der abiotischen Stresstoleranz in Kulturpflanzen.

a) Unter Hitzestressbedingungen, d.h. die getesteten (mit 2mg a.i./g Samen 4-Cyclopropylaminocarbonyl-N-(2-methoxybenzoyl)benzolsulfonamide gebeizten) Maispflanzen wurden 7 Tage nach Keimung für 6 Stunden einer Temperatur von 45˚C ausgesetzt.

Eine Durchsicht der induzierten Gengruppen ergab das folgende in Tabelle 5 dargestellte Muster

Tabelle 5

| Probe Set No. | Kondition A | Kondition B | Kondition C |
|---|---|---|---|
| Zm.11840.1.A1_at | 1.74 | 1.75 | 4.10 |
| Zm.4274.1.S1_at | 1.32 | 1.22 | 1.93 |
| Zm.3040.1.S1_at | 1.52 | 1.33 | 2.48 |
| Zm12587.1.S1.s_at | 1.30 | 1.45 | 2.33 |
| Zm18994.2.A1_a_at | 1.16 | 1.46 | 2.66 |
| Zm.13498.1.S1_at | 2.56 | 1.73 | 4.45 |

Der jeweiligen Probe Set No. entspricht:

| | |
|---|---|
| Zm.11840.1.A1_at: | Putative N-carbamyl-L-amino acid amidohydrolase |
| Zm.4274.1.S1_at: | Cytochrome P450 |
| Zm.3040.1.S1_at | Uricase II (E.C.1.7.3.3); Nodule specific uricase |
| Zm12587.1.S1.s_at: | Glycosyltransferase |
| Zm18994.2.A1_a_at: | Putative Serine Transferase |
| Zm.13498.1.S1_at: | Membrane Protein |

Kondition A: Hitzestress (6 Stunden, 45˚C)
Kondition B: 4-Cyclopropylaminocarbonyl-N-(2-methoxybenzoyl)benzolsulfonamide (VIII-3) gebeizter Samen / KEIN Hitzestress
Kondition C: 4-Cyclopropylaminocarbonyl-N-(2-methoxybenzoyl)benzolsulfonamide (VIII-3) gebeizter Samen + Hitzestress (6 Stunden, 45˚C)

Somit wurde selbst bei leichter Grundinduktion der analysierten Genaktivitäten in allen Fällen eine deutliche Steigerung der Genexpression beobachtet, die bei den hier genannten Genen im Bereich von 1.5 bis 2.35 liegt (Expression unter Kondition C / Expression unter Kondition A). Wurde die getestete Verbindung VIII-3 alleinig, d.h. ohne Hitzestress geprüft, so lagen die gemessenen Expressionslevel im Bereich des durch Hitzestress induzierten Bereichs, oder unterhalb oder leicht oberhalb des durch Hitzestress induzierten Bereichs.

Die aus der Tabelle 5 abgeleiteten Induktionsmuster, die direkt durch die erhaltenen Expressionswerte dargestellt werden, zeigen charakteristische Induktionen durch Einwirkung der Verbindung 4-Cyclopropylaminocarbonyl-N-(2-methoxybenzoyl) Benzolsulfonamide (= VIII-3), wobei die Wirkung auf das Putative N-Carbamyl-L-Amino acid Amidohydrolase [Zm.11840.1.A1_at] und auf das Putative Serine Carboxypeptidase [Zm18994.2.A1-at] am stärksten ausgeprägt ist.

b) Unter Trockenstressbedingungen, d.h. die getesteten (mit 2mg a.i./g Samen 4-Cyclopropylaminocarbonyl-N-(2-methoxybenzoyl)benzolsulfonamide gebeizten) Maispflanzen wurden 7 Tage nach Keimung für 7 Stunden einer Temperatur von 24˚C ausgesetzt.

Eine Durchsicht der induzierten Gengruppen ergab das folgende, in Tabelle 6 dargestellte, Muster

Tabelle 6

| Probe Set No. | Kondition A | Kondition B | Kondition C |
|---|---|---|---|
| Zm.818.1.A1_at | 1.06 | 1.12 | 8.47 |
| Zm.3633.4.A1_at | 1.12 | 0.74 | 3.03 |
| Zm.18273.1.S1_at | 1.66 | 0.95 | 2.91 |
| Zm.13229.1.S1_at | 1.55 | 1.02 | 3.39 |
| Zm.12035.1.A1_at | 1.86 | 0.90 | 3.66 |
| Zm.485.1.A1_at | 0.89 | 1.00 | 5.49 |

(fortgesetzt)

| Probe Set No. | Kondition A | Kondition B | Kondition C |
|---|---|---|---|
| Zm.818.2.A1_at | 0.93 | 1.10 | 5.40 |
| Zm.10097.1.A1_at | 1.23 | 1.27 | 3.29 |
| Zm.18682.1.A1_at | 1.25 | 1.12 | 4.19 |

Der jeweiligen Probe Set No. entspricht

| | |
|---|---|
| Zm.818.1.A1_at | Universal Stress Protein |
| Zm.3633.4.A1_at | Wound Induced Protein (Fragment) |
| Zm.18273.1.S1_at | Regulatory Protein-Like |
| Zm.13229.1.S1_at | NBS-LRR Type Disease Resistance Protein 02 (Fragment) |
| Zm.12035.1.A1_at | Similar to AT3G10120 |
| Zm.485.1.A1_at | Non-Symbiotic Hemoglobin (HBT) (ZEAMP GLB1) |
| Zm.818.2.A1_at | Expressed Protein |
| Zm.10097.1.A1_at | Expressed Protein |
| Zm.18682.1.A1 at | Unknown Protein |

Kondition A: Trockenstress (7 Tage, 24˚C)
Kondition B: 4-Cyclopropylaminocarbonyl-N-(2-methoxybenzoyl)benzolsulfonamide (VIII-3) gebeizter Samen / KEIN Trockenstress
Kondition C: 4-Cyclopropylaminocarbonyl-N-(2-methoxybenzoyl)benzolsulfonamide (VIII-3) gebeizter Samen + Trockenstress (7 Tage, 24˚C)

Somit wurde selbst bei leichter Grundinduktion der analysierten Genaktivitäten in allen Fällen eine deutliche Steigerung der Genexpression beobachtet, die bei den hier genannten Genen im Bereich von 1.75 bis 8.0 liegt (Expression unter Kondition C / Expression unter Kondition A). Wurde die getestete Verbindung VIII-3 alleinig, d.h. ohne Trokkenstress geprüft, so lagen die gemessenen Expressionslevel im Bereich des durch Trockenstress induzierten Bereichs, in einzelnen Fällen sogar unterhalb der Expression ungestresster Pflanzen (bei Werten < 1.0)

Die aus der Tabelle 6 abgeleiteten Induktionsmuster, die direkt durch die erhaltenen Expressionswerte dargestellt werden, zeigen charakteristische Induktionen in Gegenwart der Verbindung 4-Cyclopropylaminocarbonyl-N-(2-methoxybenzoyl) benzolsulfonamide, wobei die Wirkung auf das Universal Stress Protein [Zm.818.1.A1_at] und Non-Symbiotic Hemoglobin (HBT) (ZEAMP GLB1) [Zm.485.1.A1_at] am stärksten ausgeprägt ist.

## Patentansprüche

1. Verwendung von Verbindungen, die in ihrer Verwendung als Safener bereits im Pflanzenschutz bekannt sind, ausgewählt aus der Gruppe bestehend aus Mefenpyr-diethyl, Isoxadifen-ethyl und 4-Cyclopropylaminocarbonyl-N-(2-methoxybenzoyl)benzolsulfonamid zur Ertragssteigerung in Kulturpflanzen durch Erhöhung der Toleranz gegenüber Trocken- und/oder Hitzestress.

2. Verwendung von Verbindungen gemäß Anspruch 1, ausgewählt aus der Gruppe bestehend aus Mefenpyr-diethyl und 4-Cyclopropylaminocarbonyl-N-(2-methoxybenzoyl)benzolsulfonamid.

3. Verwendung von Verbindungen gemäß einem der Ansprüche 1 oder 2, zur Ertragssteigerung durch Toleranzerhöhung gegenüber Trocken- und/oder Hitzestress_in den Kulturpflanzen Mais, Weizen, Gerste, Roggen, Hafer, Reis, Soja, Sonnenblume, Raps und Zuckerrübe.

4. Verfahren zur Ertragssteigerung in Nutzpflanzenkulturen, die Trocken- und/oder Hitzestress ausgesetzt sind, **dadurch gekennzeichnet, dass** die Nutzpflanzen durch Saatgut-Beizung, durch Blattspritzung oder durch Bodenapplikation mit einem oder mehreren Verbindungen ausgewählt aus der Gruppe bestehen aus Mefenpyr-diethyl, Isoxadifen-ethyl, und 4-Cyclopropylaminocarbonyl-N-(2-methoxybenzoyl)benzolsulfonamid behandelt werden.

**5.** Verfahren gemäß Anspruch 4, **dadurch gekennzeichnet, dass** die Verbindung ausgewählt wird aus der Gruppe bestehend aus Mefenpyr-diethyl und 4-Cyclopropylaminocarbonyl-N-(2-methoxybenzoyl)benzolsulfonamid.

## Claims

**1.** The use of compounds whose use as safeners is already known in crop protection, selected from the group consisting of mefenpyr-diethyl, isoxadifen-ethyl and 4-cyclopropylaminocarbonyl-N-(2-methoxybenzoyl)benzenesulfonamide for increasing the yield in crop plants by increasing the tolerance to drought and/or heat stress.

**2.** The use of compounds as claimed in claim 1, selected from the group consisting of mefenpyr-diethyl and 4-cyclopropylaminocarbonyl-N-(2-methoxybenzoyl)benzenesulfonamide.

**3.** The use of compounds as claimed in either of claims 1 and 2 for increasing the yield by increasing the tolerance to drought and/or heat stress in the crop plants maize, wheat, barley, rye, oats, rice, soya, sunflower, oilseed rape and sugar beet.

**4.** A method of increasing the yield in crops of useful plants which are exposed to drought and/or heat stress, which comprises treating the useful plants by seed dressing, by foliar sprays or by soil application, with one or more compounds selected from the group consisting of mefenpyr-diethyl, isoxadifen-ethyl and 4-cyclopropylaminocarbonyl-N-(2-methoxybenzoyl)benzenesulfonamide.

**5.** The method as claimed in claim 4, wherein the compound is selected from the group consisting of mefenpyr-diethyl and 4-cyclopropylaminocarbonyl-N-(2-methoxybenzoyl)benzenesulfonamide.

## Revendications

**1.** Utilisation de composés qui sont déjà connus pour leur utilisation comme phytoprotecteurs dans la protection des plantes, choisis dans le groupe constitué par le méfenpyr-diéthyle, l’isoxadifène-éthyle et le 4-cyclopropylaminocarbonyl-N-(2-méthoxybenzoyl)benzènesulfonamide, pour l’augmentation du rendement chez des plantes cultivées, par accroissement de la tolérance au stress de sécheresse et/ou de chaleur.

**2.** Utilisation de composés selon la revendication 1, choisis dans le groupe constitué par le méfenpyr-diéthyle et le 4-cyclopropylaminocarbonyl-N-(2-méthoxybenzoyl)benzènesulfonamide.

**3.** Utilisation de composés selon la revendication 1 ou 2, pour l’augmentation du rendement par accroissement de la tolérance au stress de sécheresse et/ou de chaleur chez les plantes cultivées maïs, blé, orge, seigle, avoine, riz, soja, tournesol, colza et betterave sucrière.

**4.** Procédé pour l’augmentation du rendement dans des cultures de plantes utiles qui sont exposées au stress de sécheresse et/ou de chaleur, **caractérisé en ce qu’**on traite les plantes utiles par traitement de semences, par pulvérisation sur les feuilles ou par application sur le sol, avec un ou plusieurs composés choisis dans le groupe constitué par le méfenpyr-diéthyle, l’isoxadifène-éthyle et le 4-cyclopropyl-aminocarbonyl-N-(2-méthoxybenzoyl) benzènesulfonamide.

**5.** Procédé selon la revendication 4, **caractérisé en ce que** le composé est choisi dans le groupe constitué par le méfenpyr-diéthyle et le 4-cyclopropylaminocarbonyl-N-(2-méthoxybenzoyl)benzène-sulfonamide.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 200028055 A, Schading and Wei
- US 5201931 A, Abrams and Gusta
- DE 3534948, Draber
- DE 4103253, Bergmann
- DD 277832, Bergmann
- DD 277835, Bergmann
- WO 9916744 A
- EP 365484 A
- EP 1019368 A
- EP 0333131 A
- ZA 891960
- EP 0269806 A
- US 4891057 A
- EP 0346620 A
- AU 8934951 A
- EP 0174562 A
- WO 9108202 A
- WO 9107874 A
- WO 9507897 A
- ZA 947120
- EP 0086750 A
- EP 094349 A
- US 4902340 A
- EP 0191736 A
- US 4881966 A
- EP 0492366 A
- EP 0582198 A
- WO 200234048 A
- US 4021224 A
- US 4021229 A
- CN 87102789 A
- WO 9745016 A
- EP 0860750 A
- EP 0094349 A
- US 4137070 A
- EP 0149974 A
- HU 2143821
- EP 0221044 A
- EP 0131624 A
- WO 9211376 A
- WO 9214827 A
- WO 9119806 A
- EP 0242236 A
- EP 242246 A
- WO 9200377 A
- EP 0257993 A
- US 5013659 A
- EP 0142924 A
- EP 0193259 A
- WO 9113972 A


### In der Beschreibung aufgeführte Nicht-Patentliteratur


- **Hans W. Heldt.** Pflanzenbiochemie. Spektrum Akademischer Verlag, 1996, 393-462
- Biochemistry and Molecular Biology of Plants. American Society of Plant Physiologists, 2000, 1102-1203
- **Jaglo-Ottosen et al.** *Science,* 1998, vol. 280, 104-106
- **Hasegawa et al.** *Annu Rev Plant Physiol Plant Mol Biol,* 2000, vol. 51, 463-499
- **Ingram ; Bartels.** *Annu Rev Plant Physiol Plant Mol Biol,* 1996, vol. 47, 277-403
- **Close.** *Physiol Plant,* 1997, vol. 100, 291-296
- **Bray.** *Plant Physiol,* 1993, vol. 103, 1035-1040
- **Kirch et al.** *Plant Mol Biol,* 2005, vol. 57, 315-332
- **Yu et al.** *Mol Cells,* 2005, vol. 19, 328-333
- **Rensink et al.** *Genome,* 2005, vol. 48, 598-605
- **Cheong et al.** *Plant Physiology,* 2002, vol. 129, 661-677
- **Churchill et al.** *Plant Growth Regul,* 1998, vol. 25, 35-45
- **Bartlett et al.** *Pest Manag Sci,* 2002, vol. 60, 309
- **Morrison ; Andrews.** *J Plant Growth Regul,* 1992, vol. 11, 113-117
- **Chen et al.** *Plant Cell Environ,* 2000, vol. 23, 609-618
- **Altschul et al.** *J. Mol. Biol.,* 1990, vol. 215, 403-410
- **R. Rauhut.** Bioinformatik. Verlag Wiley-VCH Verlag GmbH, 2001, 38-107
- **R. Rauhut.** *Bioinformatik,* 2001, 197-199
- The Pesticide Manual. The British Crop Protection Council and the Royal Society of Chemistry, 1997

- **Sambrook et al.** Molecular Cloning, A Laboratory Manual. Cold Spring Harbor Laboratory Press, 1989
- **Winnacker.** Gene und Klone. VCH, 1996
- **Christou.** *Trends in Plant Science,* 1996, vol. 1, 423-431
- **Braun et al.** *EMBO J.,* 1992, vol. 11, 3219-3227
- **Wolter et al.** *Proc. Natl. Acad. Sci. USA,* 1988, vol. 85, 846-850
- **Sonnewald et al.** *Plant J.,* 1991, vol. 1, 95-106